(19) Europäisches Patentamt
European Patent Office
Office européen des brevets

(11) EP 0 515 615 B1

(12) EUROPÄISCHE PATENTSCHRIFT

(45) Veröffentlichungstag und Bekanntmachung des Hinweises auf die Patenterteilung:
**04.09.1996 Patentblatt 1996/36**

(21) Anmeldenummer: **92900474.5**

(22) Anmeldetag: **12.12.1991**

(51) Int Cl.[6]: **G01N 33/543**, G01N 33/551, G01N 33/547, G01N 21/55, G01N 27/00

(86) Internationale Anmeldenummer:
**PCT/EP91/02393**

(87) Internationale Veröffentlichungsnummer:
**WO 92/10757 (25.06.1992 Gazette 1992/14)**

(54) **UNIVERSALBINDEFILM**
UNIVERSAL BINDING FILM
PELLICULE UNIVERSELLE DE LIAISON

(84) Benannte Vertragsstaaten:
**AT BE CH DE DK ES FR GB GR IT LI LU NL SE**

(30) Priorität: **12.12.1990 DE 4039677**

(43) Veröffentlichungstag der Anmeldung:
**02.12.1992 Patentblatt 1992/49**

(73) Patentinhaber: **BOEHRINGER MANNHEIM GMBH**
**68298 Mannheim (DE)**

(72) Erfinder:
- **KNOLL, Wolfgang**
**D-6500 Mainz (DE)**
- **SCHMITT, Franz-Josef**
**D-5501 Leiwen (DE)**
- **KLEIN, Christian**
**D-8120 Weilheim (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**81635 München (DE)**

(56) Entgegenhaltungen:
**EP-A- 0 112 721**
**EP-A- 0 254 575**
**EP-A- 0 263 184**
**EP-A- 0 295 073**
**EP-A- 0 339 821**

- **BIOCHEMISTRY. Bd. 28, 1989, EASTON, PA US Seiten 8221 - 8227; R. BLANKENBURG ET AL.: 'INTERACTION BETWEEN BIOTIN LIPIDS AND STREPTAVIDIN IN MONOLAYERS: FORMATION OF ORIENTED TWO-DIMENSIONAL PROTEIN DOMAINS INDUCED BY SURFACE RECOGNITION' in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 515 615 B1

Printed by Jouve, 75001 PARIS (FR)

**Beschreibung**

Die vorliegende Erfindung betrifft eine Bindematrix, die ein Trägermaterial und einen daran über Ankergruppen adsorbierten Festphasen-Reaktanden, der mit mindestens einem freien Reaktionspartner bindefähig ist, enthält, wobei dieser Festphasen-Reaktand eine verdünnte Bindeschicht auf der Oberfläche des Trägermaterials bildet.

Bei molekularen Erkennungsreaktionen handelt es sich um die feste und spezifische Bindung zweier Moleküle, die ohne die Ausbildung einer kovalenten Atombindung eintritt. Für eine praktische Handhabung sind insbesondere solche Reaktionen von Interesse, die an der Grenzfläche zwischen einem festen Trägermaterial und einer fluiden Umgebung ablaufen. Dazu wird die Oberfläche des festen Trägermaterials mit einer Fixierschicht belegt, die einen Festphasen-Reaktanden enthält. An dieser Fixierschicht laufen dann die eigentlichen Erkennungsreaktionen ab.

Ein Beispiel einer solchen Fixierschicht ist an polymerisiertes Albumin gebundenes Streptavidin, das wiederum gut an Kunststoffoberflächen adsorptiv bindet. Diese Festphase kann mittels Bindung an Biotin bzw. an biotinylierte Reaktanden für eine große Zahl von Immuntests verwendet werden. Diese auf Streptavidin/Polyalbumin basierende Bindematrix ist sehr gut für "große" Kunststoffoberflächen geeignet. Verkleinert man jedoch die beschichtete Oberfläche, so nimmt die Genauigkeit der Tests ab. Bei neuen Testsystemen - z.B. klassischer ELISA oder Bestimmung über optische oder elektrochemische Sensoren - besteht ein wachsender Bedarf an Miniaturisierung.

In Arbeiten von Blankenburg et al. (Biochemistry 28 (1989), 8214) und Ahlers et al. (Thin Solid Films 180 (1989) 93-99) werden Streptavidin-Monoschichten beschrieben, die auf einem Langmuir-Blodgett (LB)-Film basieren. Dazu werden zunächst Biotinlipidmonoschichten mit der komplizierten Technik der Filmwaage hergestellt, die anschließend mit einer Streptavidinlösung ca. 2 Stunden lang inkubiert werden müssen. Ein Nachteil dieser LB-Filme ist außerdem ihre begrenzte Stabilität, insbesondere gegenüber Austrocknen.

Eine weitere Möglichkeit zur Herstellung einer Fixierschicht auf einem Trägermaterial ist das sogenannte Self-assembled monolayer" (SAM). So beschreiben Nuzzo und Allara (J.Am.Chem. Soc. 105 (1983), 4481-4483) die Adsorption von organischen Disulfiden auf Gold, die zu einer dichtgepackten Monoschicht führt. Die spontane Organisation solcher Monoschichten (daher die Bezeichnung SAM) beruht auf starken spezifischen Wechselwirkungen zwischen dem Trägermaterial und dem Adsorbat. Bain und Whitesides (Angew.Chem. 101 (1989), 522-528) beschreiben SAM, die durch Adsorption von langkettigen Thiolen an Gold-oberflächen entstehen. So erhält man durch Inkubation einer Goldoberfläche mit Thiolen der Formel

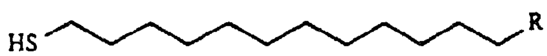

R = Alkyl, Vinyl, Halogen, Carbonsäure, Ester, Amid, Nitril, OH, Ether.

aus verdünnter organischer Lösung (z.B. 1 mmol/l) eine dichtgepackte Monoschicht. Diese Monoschichten sind im trockenen Zustand, in Wasser oder Ethanol bei Raumtemperatur über mehrere Monate stabil. Bei Erhitzen auf Temperaturen über 70°C tritt Desorption ein. Die Stabilität der Monoschicht steigt mit der Länge der aliphatischen Kette des Thiols. Auch gegen verdünnte Säuren (z.B. 1 N HCl) oder gegen verdünnte Laugen (z.B. 1 N NaOH) sind diese Monoschichten über einen gewissen Zeitraum (1 bis 7 Tage) stabil.

Die EP-A 0 339 821 offenbart Polymere für die Beschichtung von Metalloberflächen, die Thiolgruppen als Bindevermittler zum festen Trägermaterial und Aminogruppen enthalten, um einen geeigneten Festphasenreaktanden, z.B. Biotin und dann daran Streptavidin zu binden. Bei diesen Thiolgruppen-haltigen Polymeren läßt sich jedoch ebenfalls aufgrund ihrer polymeren Natur keine streng homogene Beschichtung erreichen.

Eine Arbeit von Ebersole et al. (J.Am.Chem.Soc. 122 (1990), 3239-3241) offenbart funktionell aktive Monoschichten von Avidin und Streptavidin durch direkte Adsorption dieser Proteine an Gold- und Silberoberflächen. Dabei entsteht eine dichtgepackte Streptavidin-Bindephase, die trotz einer relativ hohen Inkubationszeit von 20 Minuten mit einem biotinylierten Bindepartner nur eine sehr unvollständige Belegung mit diesem Bindepartner ergibt.

EP-A-0 295 073 offenbart ein chromatographisches Trägermaterial, worin eine Bindegruppe über einen nichtionischen, hydrophilen Spacer gebunden ist. Die Bindegruppe wird zur kovalenten Kopplung eines Festphasen-Reaktanden verwendet. Die Herstellung einer lateral homogenen und verdünnten Bindeschicht ist jedoch nicht offenbart.

Zusammenfassend ist zum Stand der Technik festzustellen, daß die dort beschriebenen Bindephasen auf Monoschichtbasis entweder langsam oder nur mit einer geringen Belegung einen freien Reaktanden binden können. Aufgabe der vorliegenden Erfindung war es daher, diese Nachteile des Standes der Technik mindestens teilweise zu beseitigen. Insbesondere sollte eine möglichst mikroskopisch homogene Universalbindephase bereitgestellt werden, die in möglichst kurzer Zeit eine möglichst große Menge eines freien Reaktanden binden kann.

Die erfindungsgemäße Aufgabe wird gelöst durch eine Bindematrix, enthaltend ein Trägermaterial und einen daran

über Ankergruppen adsorbierten Festphasen-Reaktanden, der mit mindestens einem freien Reaktionspartner bindefähig ist, wobei der Festphasen-Reaktand, der mit der Ankergruppe jeweils über ein flexibles Spacermolekül verknüpft ist, eine verdünnte lateral homogene Bindeschicht auf der Oberfläche des Trägermaterials bildet und die Bindeschicht weiterhin Spacermoleküle enthält, die mit Ankergruppen versehen sind, aber nicht mit einem Festphasen-Reaktanden verknüpft sind.

In einer bevorzugten Ausführungsform besteht die verdünnte Monoschicht, bezogen auf Festphasen-Reaktand, aus einer Molekülsorte, wobei die Oberfläche nicht vollständig belegt ist. Der Belegungsgrad mit dem Festphasenreaktanden, der ein Maß für die "Verdünnung" ist, kann ausgedrückt werden als Quotient aus gefundener Dicke der Monoschicht dividiert durch die theoretische Schichtdicke bei dichter Packung.

Der Belegungsgrad der Monoschichten mit dem Festphasen-Reaktanden ist kleiner als 100 %, vorzugsweise 0,1 bis 90 %, besonders bevorzugt 0,5 bis 70 %, am meisten bevorzugt 1 bis 40 %.

Durch den relativ großen Abstand der einzelnen Moleküle des Festphasen-Reaktanden auf der Oberfläche des Trägermaterials enthält die erfindungsgemäße Bindematrix eine aufgelockerte Schicht im Gegensatz zur dichtgepackten Schicht des Standes der Technik. Die verdünnte Monoschicht an der Oberfläche der erfindungsgemäßen Bindematrix ermöglicht eine schnellere Bindung des freien Reaktionspartners aus einer fluiden Phase und zeichnet sich überraschenderweise durch eine höhere Bindekapazität aus.

Das Trägermaterial der erfindungsgemäßen Bindematrix kann eine Metall-, Metalloxid- oder Glasoberfläche aufweisen. Vorzugsweise besitzt das Trägermaterial eine Metalloberfläche, besonders bevorzugt eine Edelmetallobefläche. Die Herstellung eines Trägermaterials mit einer Goldoberfläche geschieht beispielsweise durch Bedampfen von Glas mit Chrom als Haftvermittler, wobei eine ca. 0,1 bis 10 nm dicke Schicht entsteht. Diese Chromschicht wird anschließend mit Gold bedampft, wobei eine Goldschicht entsteht, welche die Oberfläche des Trägermaterials für eine erfindungsgemäße Bindematrix darstellt. Diese Goldschicht ist zweckmäßig zwischen ca. 10 und 100 nm dick, wenn die Bindematrix im Rahmen der Oberflächenplasmonenresonanz verwendet wird. Bei anderen Anwendungen, z.B. als elektrochemischer Sensor, kann die Bindematrix auch dicker gewählt werden.

Die Adsorption des Festphasen-Reaktanden an die Oberfläche des Trägermaterials wird durch Ankergruppen vermittelt. Die Art der Ankergruppen hängt von der jeweiligen Oberfläche des Trägermaterials ab. Als Ankergruppen für ein Trägermaterial mit einer Metalloberfläche sind Thiol-, Disulfid- oder Phosphingruppen geeignet. So sind z.B. Thiol- oder Disulfidgruppen als Ankergruppen für Gold- oder Silberoberflächen und Phosphingruppen für eine Palladiumoberfläche besonders geeignet. Weist das Trägermaterial eine Metalloxidoberfläche (z.B. $Al_2O_3$ ) auf, so ist als Ankergruppe eine Carboxylat- oder Sulfonatgruppe geeignet. Bei einer Glas/Siliciumoberfläche und hydroxylierten Oberflächen, wie $SiO_2$ auf Si, $Al_2O_3$ auf Al verwendet man als Ankergruppen Organosilicium-Verbindungen. Vorzugsweise werden Trichlorsilane verwendet (Sagiv, J.Amer.Chem.Soc. 102 (1980) 82).

Die Ankergruppe für die Adsorption ist an die Festphase nicht direkt am Festphasen-Reaktanden selbst angebracht, sondern über ein flexibles Spacermolekül mit dem Festphasen-Reaktanden verknüpft. Besonders bevorzugt enthält das Spacermolekül mindestens eine Alkylengruppe der Formel $(CH_2)_n$, worin n eine natürliche Zahl von 1 bis 30, vorzugsweise 2 bis 30, besonders bevorzugt 2 bis 15 darstellt. Auf seiner einen Seite enthält das Spacermolekül die Ankergruppe (z.B. die Thiol-oder Disulfidgruppe), die zur Adsorption an die Oberfläche des Trägermaterials geeignet ist. Auf seiner anderen Seite, d.h. vom Trägermaterial wegstehend enthält das Spacermolekül eine oder mehrere Verknüpfungsgruppierungen, über die der Festphasen-Reaktand oder eine Komponente davon mit dem Spacermolekül verknüpft ist. Bei diesen Verknüpfungsgruppierungen kann es sich z.B. um eine Amino- oder Hydroxylfunktion handeln, die z.B. mit einer Carboxylfunktion des Festphasen-Reaktanden unter Bildung einer Ester- oder Amidgruppe verknüpft ist. Das Spacermolekül kann jedoch auch als Verknüpfungsgruppe eine Carboxylfunktion enthalten, die dann wiederum mit einer reaktiven Amino- oder Hydroxylfunktion des Festphasenreaktanden verknüpft ist.

Es soll klargestellt werden, daß für die Herstellung einer erfindungsgemäßen Bindematrix mit einer verdünnten Monoschicht des Festphasen-Reaktanden mehrere Möglichkeiten bestehen. Im folgenden werden einige dieser Möglichkeiten aufgeführt, diese Aufzählung soll jedoch den Umfang der vorliegenden Erfindung nicht darauf beschränken.

Zunächst soll jedoch eine nicht erfindungsgemäße, dichtgepackte Monoschicht eines Festphasen-Reaktanden beschrieben werden. Eine solche Schicht ist erhältlich, wenn man ein Lipid gemäß der Arbeit von Bain und Whitesides (Angew. Chem. 101 (1989), 522-528), das eine Hydroxyl- oder Aminoendgruppe aufweist, mit einem aktivierten Biotinderivat umsetzt, wobei unter Verwendung von 11-Hydroxy-undekan-1-thiol als Ausgangsmaterial ein biotinyliertes Lipid mit folgender Formel entsteht:

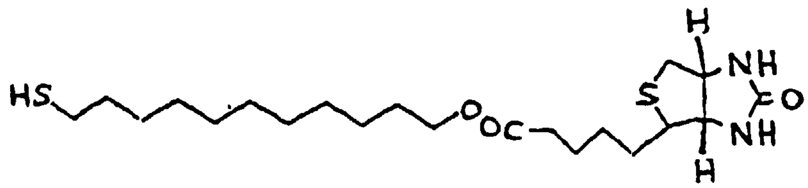

Bei Adsorption dieses Lipids an ein Trägermaterial mit Goldoberfläche bis zur Sättigung entsteht gemäß Dickenmessung eine dicht gepackte Monoschicht mit einer Belegung von 100 % bezogen auf Biotin. Auf diese Weise erhält man eine starre Bindematrix, die als solche nicht erfindungsgemäß ist und auch eine nur geringe Bindekapazität für einen freien Reaktionspartner (in diesem Falle Streptavidin) besitzt.

Im Gegensatz dazu ist die Herstellung einer erfindungsgemäßen Bindematrix durch Verwendung eines Spacermoleküls möglich, das mit zwei oder mehreren Molekülen, vorzugsweise 2 Molekülen des Festphasen-Reaktanden gleichzeitig verknüpft ist. Ein Beispiel für ein solches Spacermolekül ist Cystamin, das als Ankergruppe eine Disulfidgruppe und als Verknüpfungsgruppierung zwei Aminofunktionen enthält und daher mit zwei Molekülen eines aktivierten Biotins verknüpft werden kann, wobei ein biotinyliertes Lipid mit folgender Formel entsteht:

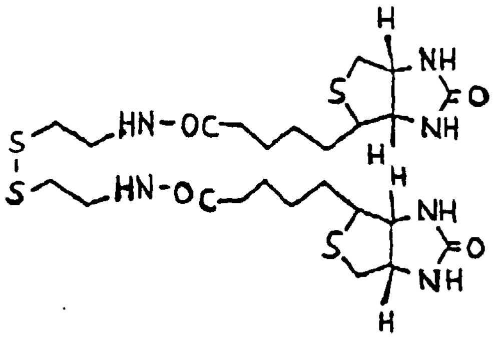

Dieses biotinylierte Lipid bildet bei Adsorption an eine Goldoberfläche eine erfindungsgemäße Bindematrix mit einem Belegungsgrad von 30 % bezogen auf Biotin aus, die einen freien Reaktionspartner (Streptavidin) mit hoher Affinität zu einem dichten Film binden kann.

Eine weitere Möglichkeit zur Herstellung einer erfindungsgemäßen Bindematrix ist der Einbau einer hydrophilen Linkergruppe zwischen dem Spacermolekül und der Festphasen-Reaktanden. Dieser Linker ist insbesondere ein geradkettiges Molekül mit einer Kettenlänge von 4 bis 15 Atomen. Bevorzugt ist dabei eine Linkergruppe, die eine oder mehrere hydrophile Ethylenoxideinheiten, vorzugsweise zwischen 1 und 5 enthält. Besonders bevorzugt wird die hydrophile Linkergruppe durch ein Amin- oder Hydroxyl-terminiertes Polyethylenoxid gebildet.

Zwischen dem hydrophilen Linker und dem Festphasen-Reaktanden kann vorzugsweise ein weiteres Spacermolekül eingebaut werden, welches aus einer Alkylengruppe der Formel $(CH_2)_n$ und einer Verknüpfungsgruppierung besteht, worin n eine natürliche Zahl von 2 bis 15 ist.

Als besonders geeigneter Linker hat sich 1,8-Diamino-3,6-dioxaoctan erwiesen. So entsteht durch den Einbau von 1,8-Diamino-3,6-dioxaoctan zwischen ein $C_{11}$ -Thiolspacermolekül und Biotin eine biotinylierte Verbindung der folgenden Formel:

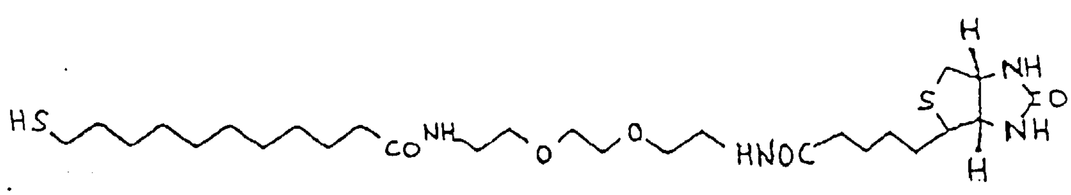

Diese Verbindung bildet bei Adsorption an eine Goldoberfläche eine Monoschicht mit einer Belegungsdichte von 19 % bezogen auf Biotin aus, die in der Lage ist, einen freien Reaktionspartner (Streptavidin) mit hoher Affinität und innerhalb kürzester Zeit zu einem dichtgepackten Film zu binden. Eine erfindungsgemäße Bindematrix, in der ein Spacermolekül über eine hydrophile Linkergruppe mit dem Festphasen-Reaktanden verknüpft vorliegt, ist daher im Sinne der vorliegenden Erfindung besonders bevorzugt.

Die erfindungsgemäße Bindematrix enthält zusätzlich noch Spacermoleküle, die zwar mit einer Ankergruppe versehen sind, aber an die kein Festphasen-Reaktand gebunden ist. Derartige Verbindungen werden im weiteren auch

als Verdünnungsmoleküle bezeichnet. Wenn man z.B. biotinylierte und nicht biotinylierte Spacermoleküle im Verhältnis von 1:10 bis 1:2 einsetzt, so erhält man eine verdünnte Biotin-Monoschicht, die den freien Reaktionspartner mit hoher Geschwindigkeit und großer Kapazität binden kann.

Geeignete Verdünnungsmoleküle enthalten eine Ankergruppe und einen Spacerbestandteil sowie ggf. ein Linkermolekül, wobei sich die Anzahl der $CH_2$-Gruppen des Spacermoleküls um nicht mehr als 1 - 5, bevorzugt nicht mehr als 1 - 2 C-Atome von der Anzahl der $CH_2$-Gruppen des Spacermoleküls unterscheidet, das an den Festphasen-Reaktanden gebunden vorliegt. Es hat sich weiterhin als zweckmäßig erwiesen, daß die Mindestkettenlänge des Verdünnungsmoleküls 6 Atome (ohne Ankergruppe und hydrophile Linkergruppe) beträgt.

Anstelle des Festphasen-Reaktanden befindet sich vorzugsweise an dem von der Ankergruppe entfernten Ende des Verdünnungsmoleküls eine hydrophile Funktion, wie z. B. eine Hydroxylgruppe, eine Carbonsäuregruppe, eine Carbonsäureethylester- oder Methylestergruppe, eine Carbonsäureamidgruppe, eine mit 1 oder 2 Methyl- oder Ethylgruppen substitutierte Carbonsäureamidgruppe, eine Sulfonsäuregruppe oder eine Sulfonamidgruppe. Ebenso ist es bevorzugt, an das von der Ankergruppe entfernte Ende des Verdünnungsmoleküls einen hydrophilen Linker (gemäß obiger Definition) oder einen Teil eines hydrophilen Linkers zu binden. Folglich enthält ein bevorzugtes Verdünnungsmolekül auf der einen Seite des Spacerbestandteils eine mit dem Trägermaterial reaktive Ankergruppe und auf der anderen Seite eine hydrophile Endgruppe.

In einer weiteren Ausführungsform der Erfindung können ein Spacer mit Festphasen-Reaktand und ein Spacer ohne Festphasen-Reaktand über eine kovalente Bindung verknüpft. Bei Verwendung von Gold- oder Silberoberflächen erfolgt diese Verknüpfung vorzugsweise über eine Disulfidbrücke.

In solchen gemischten Monoschichten, die aus Verdünnungsmolekülen (Spacermolekülen ohne Festphasen-Reaktand) und aus Spacermolekülen mit Festphasen-Reaktand bestehen, beträgt der Anteil der Spacermoleküle mit Festphasen-Reaktand zweckmäßig 0,1 - 90 mol-%, vorzugsweise 0,5 - 50 mol-% und besonders bevorzugt 1 - 40 mol-%.

In allen bisher genannten Bindefilmen besteht der Festphasen-Reaktand aus einer Komponente. Dabei handelt es sich vorzugsweise um Biotin oder Biotin-analoge Moleküle wie Desthiobiotin, Iminobiotin oder HABA (4-Hydroxyphenyl-azobenzoesäure), die ebenfalls mit Streptavidin reagieren.

Weitere Beispiele für geeignete Festphasen-Reaktanden sind jedoch auch mit einem Antikörper bindefähige Antigene oder Haptene. In diesem Fall ist der Festphasen-Reaktand vorzugsweise ein Hapten mit einem Molekulargewicht von 100 bis 1200. Geeignet sind beispielsweise Steroide (wie z.B. Digoxin, Digoxigenin, Cortisol, Oestriol, Oestradiol, Theophyllin, Diphenylhydantoin, Testosterol, Gallensäuren, Progesteron und Aldosteron); kurzkettige Peptide (wie z.B. Argipressin, Oxytocin und Bradykinin); Fluorescein und seine Derivate; $T_3$, $T_4$, Aflatoxin, Atrazin, Pflanzenhormone wie z.B. Gibberilline; und Alkaloide (wie z.B. Reserpin und Ajmalicin).

Besonders bevorzugt werden als Hapten Biotin und Biotinderivate, Digoxin, Digoxigenin, Fluorescein und Derivate sowie Theophyllin eingesetzt.

Andererseits kann der Festphasen-Reaktand auch aus mehreren Komponenten bestehen. Darunter ist insbesondere zu verstehen, daß eine innere Komponente des Festphasen-Reaktanden kovalent mit einem Spacermolekül verknüpft ist und nicht kovalent an die äußere Komponente des Festphasen-Reaktanden gebunden ist. Dabei ist dann die äußere Komponente des Festphasen-Reaktanden zur Bindung eines freien Reaktionspartners in der Lage. Beispielsweise kann es sich bei der inneren Komponente um Biotin und bei der äußeren Komponente um Streptavidin handeln. Eine solche Bindematrix ist wiederum in der Lage, biotinylierte Reaktionspartner aus einer Lösung zu binden, da Streptavidin vier Bindestellen für Biotin besitzt, von denen mindestens zwei noch frei sind.

Eine Bindeschicht, die einen aus zwei Komponenten bestehenden Festphasen-Reaktanden enthält, ist dann eine erfindungsgemäße Bindematrix, wenn die äußere, d.h. die mit einem freien Reaktionspartner bindefähige Komponente des Festphasen-Reaktanden (d.h. im speziellen Fall Streptavidin) eine verdünnte Schicht an der Oberfläche der Bindematrix bildet. Vorzugsweise bildet die innere Komponente des Festphasen-Reaktanden eine unverdünnte Schicht an der Oberfläche der Bindematrix, an die sich die äußere Komponente des Festphasen-Reaktanden unter Bildung einer verdünnten Schicht anlagern kann.

So bindet eine dichtgepackte Biotinmonoschicht mit 100 % Belegung (die selbst keine erfindungsgemäße Bindematrix darstellt) Streptavidin mit einer Belegungsdichte von 27 %. Diese verdünnte Streptavidin-Schicht stellt dann wiederum eine erfindungsgemäße Bindematrix dar, die einen freien Reaktionspartner, z.B. einen biotinylierten Antikörper zu einem dichtgepackten Film binden kann. Eine verdünnte Biotinmonoschicht, die beispielsweise durch Verwendung eines Festphasen-Reaktanden mit Spacer und Linker hergestellt wurde (vgl. Beispiel 8) und die eine Bindematrix für die Bindung von freiem Streptavidin darstellt, kann Streptavidin zu einem dichtgepackten Film mit 100 % Belegung binden. Die dabei resultierende, dichtgepackte Streptavidinschicht stellt jedoch wiederum keine erfindungsgemäß Bindematrix dar (weil nicht flexibel) und kann einen Reaktionspartner (z.B. einen biotinylierten Antikörper) nur zu einem ca. 10 % belegten Film binden.

Dieses erfindungsgemäße Prinzip des verdünnten bindefähigen Festphasen-Reaktanden läßt sich von der Biotin-Streptavidin-Bindung auch auf andere Bindepaare, also z.B. Antikörper-Antigen etc. erweitern.

Der Belegungsgrad des Festphasen-Reaktanden an der Oberfläche der Bindematrix ist durch eine Dickenmessung der Bindeschicht bestimmbar. Dabei nimmt die gemessene Schichtdicke mit dem Belegungsgrad der Bindeschicht ab. So hat eine unten beschriebene Bindeschicht mit Biotin als Festphasen-Reaktand eine Dicke von 0,7 nm, wobei diese Dicke geringer als die errechnete Länge von 3,7 nm des Moleküls ist. Ebenfalls wird eine Bindeschicht mit Streptavidin als Festphasenreaktand beschrieben, deren Dicke mit 0,5 nm weit unter dem Durchmesser des Streptavidinmoleküls liegt.

Weiterhin ein Gegenstand der vorliegenden Erfindung ist ein Verfahren zur Herstellung einer erfindungsgemäßen Bindematrix. Aufgrund der unterschiedlichen Natur der erfindungsgemäßen Bindematrizes sind auch die Herstellungsverfahren im Detail unterschiedlich. Mehrere bevorzugte Varianten dieser Herstellungsverfahren sind in den Ausführungsbeispielen dargestellt. Allgemein beinhaltet das erfindungsgemäße Verfahren die Inkubation des Trägermaterials mit einer Reaktionslösung, in der die Moleküle vorliegen, welche die Bindeschicht der erfindungsgemäßen Bindematrix bilden. Diese Moleküle enthalten an gegenüberliegenden Seiten die Ankergruppe und den Festphasen-Reaktanden, wobei nicht alle Moleküle der Bindeschicht mit einem Festphasen-Reaktanden verknüpft sind.

Der Festphasen-Reaktand ist über ein Spacermolekül mit der Ankergruppe verknüpft. Die Anlagerung der Ankergruppen an das Trägermaterial aus der Lösung unter Bildung der erfindungsgemäßen Bindematrix ist ein spontaner Prozeß.

Die Inkubation des Trägermaterials mit der Reaktionslösung zur Herstellung der ersten Bindeschicht findet vorzugsweise unter Schutzgas und zweckmäßigerweise in einem inerten Lösungsmittel, das vorzugsweise frei von störenden Substanzen, z.B. $H_2O$ oder $O_2$ ist, statt.

Gegebenenfalls kann in einem zweiten Schritt durch Inkubation mit einer zweiten Reaktionslösung eine weitere Substanz angelagert werden, insbesondere wenn der Festphasen-Reaktand aus mehreren, nicht kovalent miteinander verbundenen Komponenten besteht. Zum Aufbringen der zweiten und ggf. weiteren Schichten sind die Reaktionsbedingungen nicht kritisch, so daß ohne Schutzgas und mit Wasser als Lösungsmittel gearbeitet werden kann.

Die nach dem erfindungsgemäßen Verfahren hergestellte laterale Bindeschicht ist mikroskopisch homogen, wie z.B. durch die Surface plasmon microscopy nachweisbar (B. Rothenhäusler und W. Knoll, Surface Plasmon Microscopy, Nature, Vol. 332, Nr. 6165, S. 615-617 (1988); W. Hickel, B. Rothenhäusler und W. Knoll, "Surface Plasmon Microscopic Characterisation of external surfaces", J.Appl.Phys. (1989), S. 4832-4836; W. Hickel, W. Knoll "Surface Plasmon optical characterisation nof lipid monolayers at 5 µm lateral resolution", J.Appl.Phys. 67 (1990), S. 3572 ff.). Bei einer Auflösung von 5 µm sind keine Dickenunterschiede meßbar.

Weiterhin betrifft die vorliegende Erfindung ein Verfahren zur Bestimmung eines Analyten in einer Probelösung mittels einer spezifischen Bindungsreaktion zwischen mindestens zwei bioaffinen Reaktanden, von denen einer an eine Festphase gekuppelt vorliegt und der oder die anderen Partner frei sind, wobei man einen Festphasen-Reaktanden verwendet, der Bestandteil einer erfindungsgemäßen Bindematrix ist.

Bei einem derartigen Verfahren kann der Nachweis der Bindung des freien Reaktionspartners an den Festphasenreaktanden dadurch ermöglicht werden, daß der freie Bindungspartner eine Markierungsgruppe trägt. Gebräuchlich ist insbesondere die Markierung mit einem Enzym oder mit einem fluoreszierenden oder lumineszierenden Bestandteil. Die dadurch ermöglichte indirekte optische Beobachtung der Bindung erlaubt einen genauen quantitativen Nachweis.

Grundsätzlich kann die Bindung optisch, elektrochemisch, aber auch über die Wärmetönung oder die Massenbildung bestimmt werden. Für elektrochemische Techniken kommen insbesondere potentiometrische und amperometrische Methoden in Frage, wie sie beispielsweise in "Biosensors", Turner, Karube, Wilson (eds.), Oxford Press, 1987 oder Bergveld, Biosensors & Bio-electronics 6, 55-72 (1991) beschrieben sind. Bestimmungen über die elektrische Leitfähigkeit oder Kapazitätsänderung sind als elektrochemische Techniken ebenfalls möglich.

Vorzugsweise erfolgt jedoch der Nachweis der Bindung durch optische, insbesondere reflexionsoptische Techniken, bei der die Zunahme der Schichtdicke einer extrem dünnen Schicht mit dem trägerfixierten Reaktanden durch Bindung des freien Bindungspartners beobachtet werden kann. Ein Überblick über diese Techniken wird gegeben in Sadowski: "Review of optical methods in immunosensing", SPIE, Vol. 954 Optical testing and Metrology II (1988), 413-419.

Ein besonders bevorzugtes reflexionsoptisches Verfahren ist der Nachweis der Bindung durch Oberflächenplasmonenresonanz. Bei diesem Verfahren besteht das Analyseelement aus einem durchsichtigen dielektrischen Material, auf dem in sehr geringer Schichtdicke eine metallisch leitende Schicht aufgebracht ist, welche den Festphasen-Reaktanden trägt. Dieses Analyselement wird vielfach auch als optischer Immunsensor bezeichnet. Beispiele für solche optischen Immunsensoren sind in der EP-A 0 112 721, der EP-A 0 276 142 und in der EP-A 0 254 575 beschrieben. Besonders bevorzugt für den quantitativen Nachweis der Bindung an die Festphase ist jedoch der in Beispiel 4 dargestellte Immunsensor. Das Prinzip eines derartigen Immunsensors wird detailliert in der DE 40 24 476 vorgeschlagen, auf deren Offenbarung hier Bezug genommen wird.

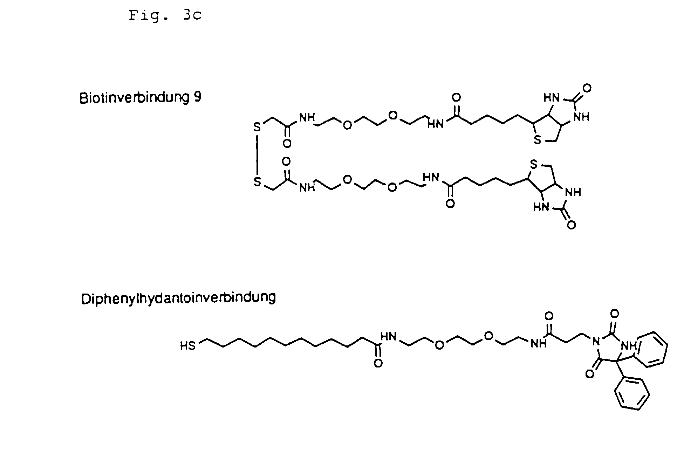

Die vorliegende Erfindung soll im weiteren durch die folgenden Beispiele in Verbindung mit den Figuren 1 bis 3 verdeutlicht werden.

Fig. 1 ist die schematische Darstellung einer Meßeinrichtung, mit der die Bindung eines freien Reaktionspartners an die Festphase bestimmt werden kann.

Fig. 2 zeigt die Dicke von Bindematrices, hergestellt durch die Kombination einer Komponente mit Festphasen-Reaktanden und eines Verdünnungsmoleküls sowie die Dicke des daran gebundenen Streptavidins (Beispiel 23 und 24):

Kurve 1: Dicke der Biotinmonolayer
Kurve 2: Dickenzuwachs durch Streptavidin in Abhängigkeit vom Molenbruch x an Biotin

$$x = \frac{C_{\text{Biotinverbindung 5}}}{C_{\text{Biotinverbindung 5}} + C_{\text{Verdünnungsmoleküle}}} : 2$$

C: molare Konzentration

Fig. 3 zeigt eine Liste der erfindungsgemäß hergestellten Biotin- oder Biotinderivatverbindungen.

Beispiel 1

Synthese von Bisbiotinoylcystamin (Biotinverbindung I)

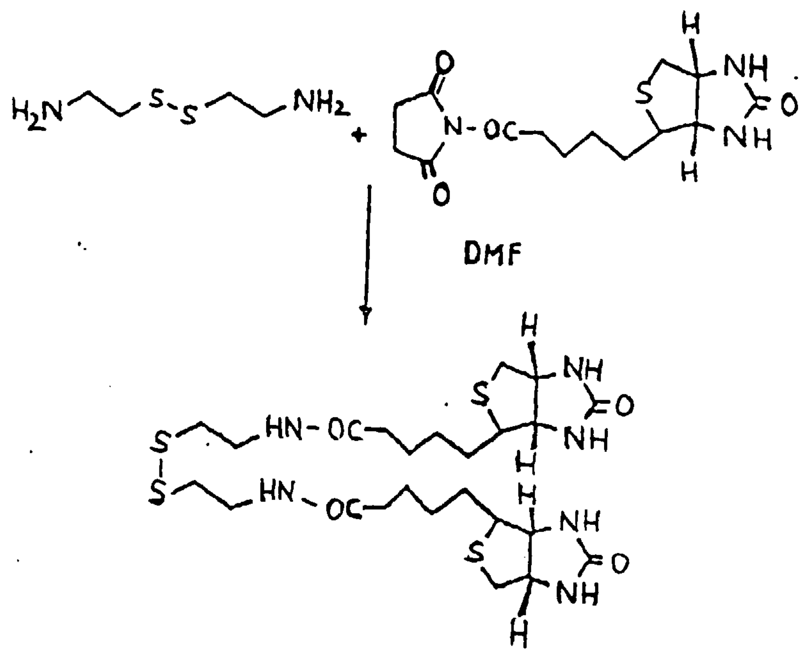


Biotin-N-hydroxysuccinimidester wurde zu einer Lösung von Cystaminiumdichlorid und Triethylamin in Dimethylformamid (DMF) gegeben. Die Reaktionsmischung wurde über Nacht bei Raumtemperatur gerührt. Nach beendeter Reaktion wurde der entstandene Niederschlag abfiltriert, an der Ölpumpe getrocknet und aus Aceton umkristallisiert. Die Zielverbindung wurde in 40 %iger Ausbeute erhalten.

Beispiel 2

Synthese von Biotin (11-mercapto)undecanylester (Biotinverbindung 2)

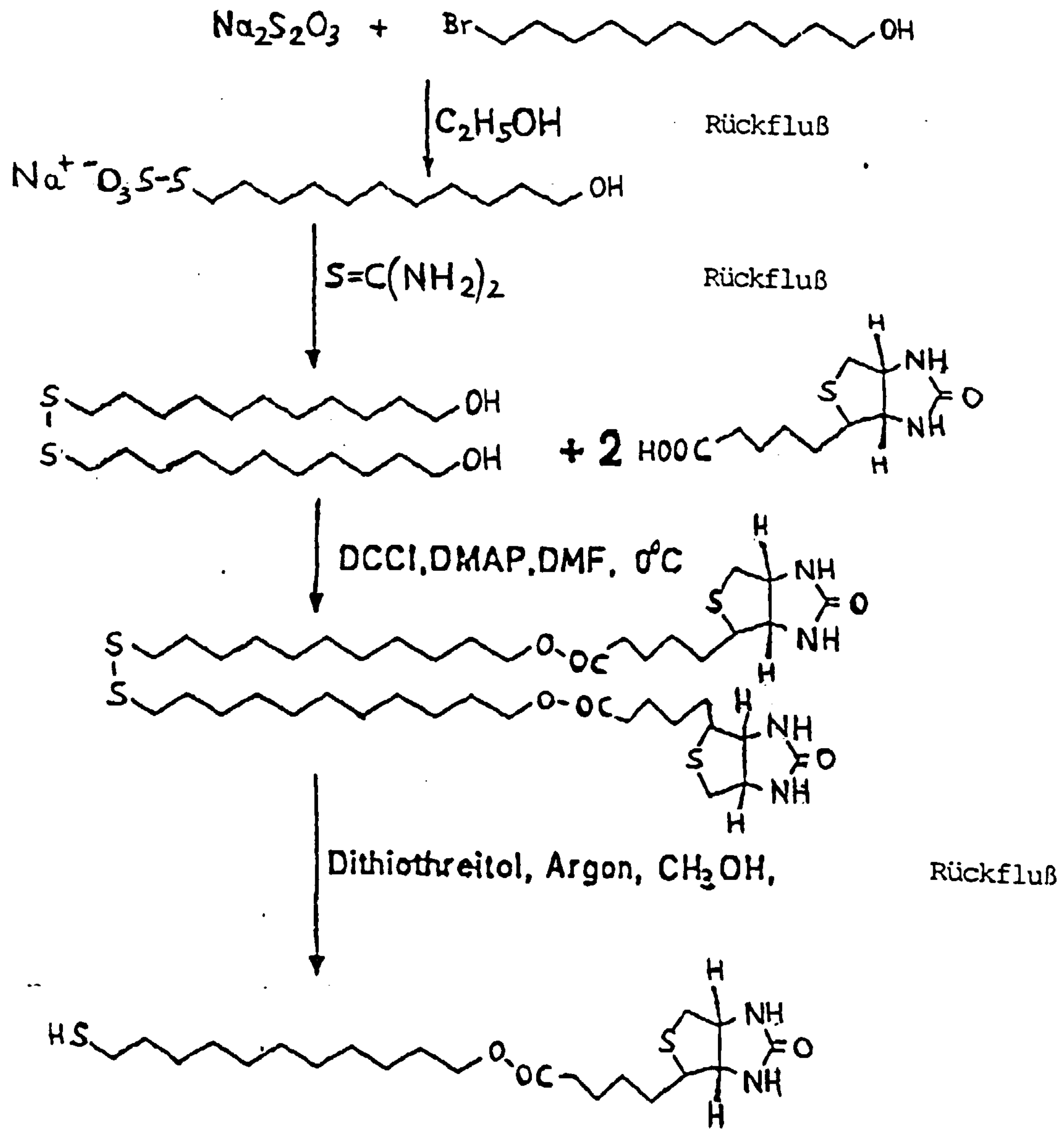


Aus Bromundecanol (Merck) wurde durch 4-stündiges Kochen der ethanolischen Lösung am Rückfluß und langsames Zutropfen einer gesättigten wäßrigen Natriumthiosulfatlösung das entsprechende Buntesalz dargestellt. Es kristallisierte beim Abkühlen der Lösung aus und wurde durch Umkristallisation aus Ethanol gereinigt.

Aus diesem Buntesalz wurde durch Kochen mit einer wäßrigen Thioharnstofflösung, die mit Salzsäure auf pH 1, gestellt worden war, das symmetrische Disulfid in 98 %iger Ausbeute dargestellt. Es kristallisierte beim Abkühlen aus der Reaktionsmischung aus.

Das Disulfid wurde mit Biotin und 10 mol% 4-Dimethylaminopyridin (DMAP) in DMF durch Erwärmen gelöst und durch Zugabe von in DMF gelöstem Dicyclohexylcarbodiimid (DCCI) die Veresterung bei -15°C eingeleitet. Nach 5 Stunden wurde der Ansatz auf Raumtemperatur gebracht und weitere 18 Stunden gerührt. Nach beendeter Reaktion wurde das DMF im ölpumpenvakuum bei Raumtemperatur entfernt und der Rückstand durch Flash-Chromatographie gereinigt. Es wurde 11,11'-Bis-Biotinylester-undecyl-1,1'-disulfid in 13 %iger und das entsprechende monosubstituierte Produkt in 23 %iger Ausbeute erhalten.

Das biotinylierte Disulfid wurde nun mit Dithiothreitol (DTT) (Aldrich) in siedendem Methanol unter Argonatmosphäre reduziert. Nach beendeter Reaktion (24 h) wurde die Reaktionsmischung bis zur Trockne eingeengt und das entstandene Disulfidothreitol sowie überschüssiges DTT durch Extraktion mit wenig Wasser abgetrennt. Das Gemisch wurde dann durch Flash-Chromatographie aufgetrennt und das gewünschte Produkt in 80 %iger Ausbeute isoliert.

Beispiel 3

Synthese von 11-Mercaptoundecansäure-(8-biotinoylamido-3,6-dioxyoctyl)amid (Biotinverbindung 3)

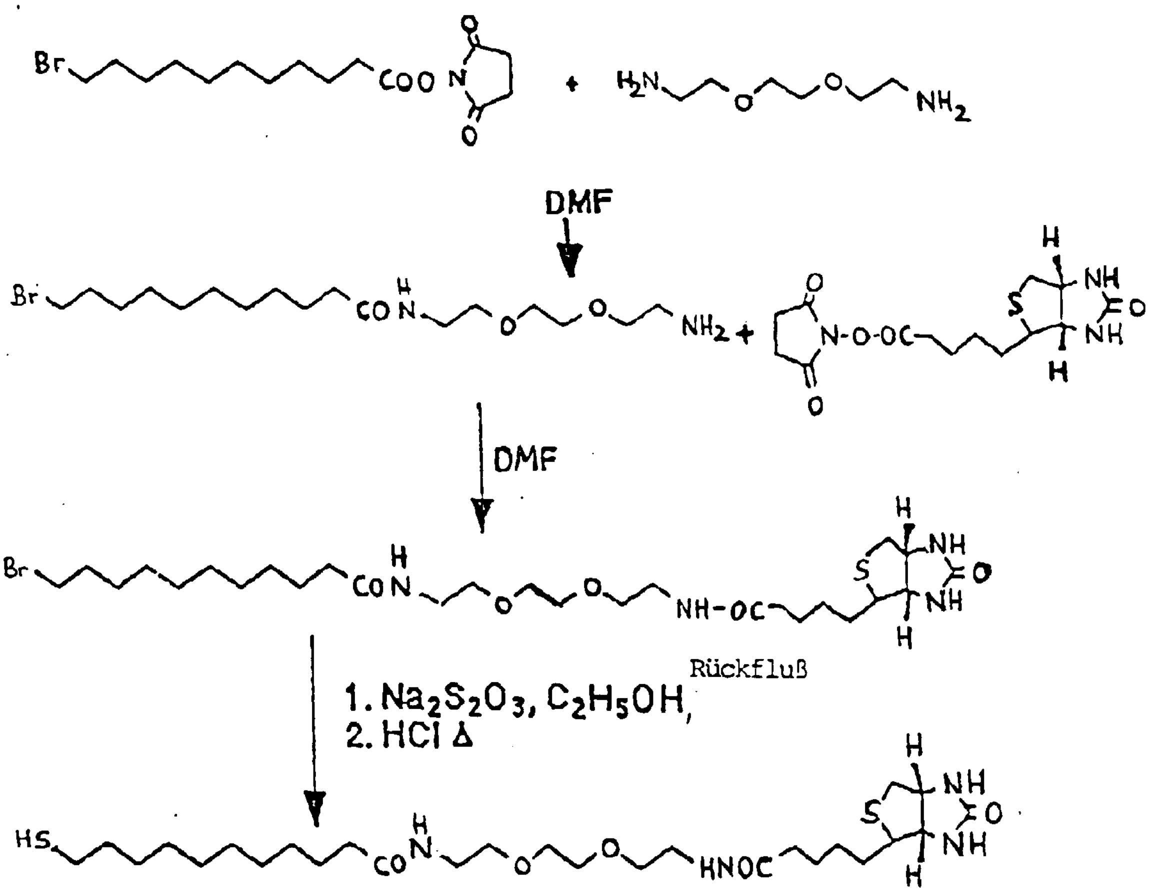


Der Aktivester der 11-Bromundekansäure wurde mit 1,8-Diamino-3,6-dioxaoctan im Überschuß in DMF-Lösung in der Kälte umgesetzt. Nach beendeter Reaktion wurde das ausgefallene N-Hydroxysuccinimid abfiltriert und das überschüssige Amin gemeinsam mit dem DMF im Ölpumpenvakuum abgetrennt.

Die erhaltene Zwischenstufe wurde dann mit Biotinaktivester, wiederum in DMF, umgesetzt. Das entstandene Alkylbromid wurde nach beendeter Reaktion durch Flash Chromatographie ($CHCl_3/CH_3OH$:1/1) isoliert.

Das Rohprodukt wurde zum Buntesalz umgesetzt (s. Beispiel 2) und anschließend direkt mit 1N Salzsäure unter Argon hydrolysiert. Die Lösung wurde heiß filtriert, um den entstandenen Schwefel abzutrennen. Daraufhin wurde das Filtrat bis zur Trockne eingeengt und das rohe Mercaptan mit Chloroform/Ethanol extrahiert, der Extrakt eingeengt und mittels Flash-Chromatographie ($CHCHl_3/CH_3OH$:1/1) abgetrennt. Das gewünschte Produkt wurde in 3 %iger Ausbeute erhalten.

Beispiel 4:

Herstellung des Biotinmonolayers 1 aus der Biotinverbindung 1

Als feste Träger dienen Objektträger aus Hochindexglas SF57, die mit Chrom (1 nm) als Haftvermittler und mit Gold (41 nm) bedampft werden. Das biotinylierte Disulfid (aus Beispiel 1) wird als $6x10^{-4}$ mol/l (M) Lösung in $CHCl_3$/EtOH 1:1 angesetzt. Unter einer Argon-Schutzgasatmosphäre wird der Objektträger zwei Stunden lang in der Reaktionslösung inkubiert, anschließend mit reinem Lösungsmittel gründlich gespült und in einem Argon-Strom getrocknet. Eingebaut in einen modifizierten Kretschmann Aufbau (Fig.1) kann die beschichtete Probe sowohl gegen Luft als auch gegen wäßrige Medien charakterisiert werden.

Figur 1 zeigt schematisch eine Meßeinrichtung zur reflexions-optischen Bestimmung der Bindung des freien Reaktionspartners an die Festphase.

Diese reflexionsoptische Meßeinrichtung enthält einen Laser 10. Der von dem Laser ausgehende Primärstrahl 11 fällt unter einem Winkel υ zur Flächennormalen 17 des Testbereichs 20 ein. Das reflektierte Licht wird von einer Sammellinse 12 auf die in der Bildebene angeordnete Diode 13 abgebildet.

Die Meßeinrichtung enthält weiterhin ein Prisma 16 in Kretschmann-Anordnung und eine Flußküvette 14 mit Zutritts-und Ausgangsöffnungen 15 für die Testlösung.

Der Testbereich 20 besteht aus einem Prisma 16 in Kretschmann-Anordnung, einer dielektrischen, optisch transparenten Trägerschicht 22 und einer dünnen Metallschicht 23a, 23b, die auf die Trägerschicht 22 aufgedampft ist. Die dünne Schicht der Indexflüssigkeit 21 verbindet das Prisma 16 ohneoptische Brechung mit der optisch transparenten Trägerschicht 22, da sie den gleichen Brechungsindex wie diese beiden Teile besitzt. Im vorliegenden Falle stellt 23a die oben erwähnte Chromschicht und 23b die die aufgedampfte Goldschicht dar. 25 stellt das Spacermolekül dar, welches über Ankergruppen die Bindung des Festphasen-Reaktanden 26 an die Goldoberfläche vermittelt. 27 stellt den freien Reaktionspartner dar, der mit dem Festphasenreaktanden bindefähig ist und in der Test-phase 28 vorliegt.

Mit Hilfe der Fresnel'schen Gleichungen läßt sich die Reflexion an Grenzflächen berechnen und an die PSP-Spektroskopie Daten anfitten, wobei man für jede Schicht eine Angabe zur "optischen Dicke" (=$n^2$xd, mit n = Brechungsindex und d = Dicke) erhält. Unter der Annahme eines Brechungsindex für Thiolalkane von n = 1,45 ergibt sich für das adsorbierte Disulfid eine Dicke von 0,5 nm. Vergleiche mit dem theoretischen Wert von -1,6 nm deuten auf eine bei weitem nicht vollständige Belegung der Oberfläche hin.

Mit einer ca. 30 %igen Belegung der Oberfläche liegt daher ein "verdünnter" Biotinfilm vor.

Beispiel 5

Bindung von Streptavidin an den Biotin-Monolayer 1/ Herstellung des Streptavidin-Monolayers 1

Um die Reaktionsfähigkeit der biotinylierten Fixierschicht zu überprüfen, tauscht man die 0,5 M wäßrige NaCl-Lösung (als Referenz) gegen eine ~5x$10^{-7}$ M Streptavidin-Lösung aus.

Die anschließende molekulare Erkennungsreaktion durch Streptavidin erreicht innerhalb einer Stunde den Sättigungswert von 3,0 nm Dickenzunahme. Dabei wurde ein Brechungsindex des Proteins von n = 1,5 zugrunde gelegt. Es ist anzunehmen, daß der wahre, optisch effektive Brechungsindex niedriger liegt, da die Zwischenräume der adsorbierten Proteinmoleküle mit Wasser (n = 1,33) gefüllt sind. Unter Berücksichtigung dieser Erkenntnis läßt sich der gefundene Dickenzuwachs recht gut mit den röntgenographischen Werten von einkristallinem Streptavidin (d = ~4,5 nm) vergleichen.

Beispiel 6

Herstellung des Biotin-Monolayers 2 aus der Biotinverbindung 2

Die Präparation erfolgte größtenteils analog zu Beispiel 4. Abweichungen traten nur bei der Thiol-Adsorption an den festen Trägern auf. So betrug die Konzentration der Lösung hier 4x$10^{-4}$ mol/l und die Inkubation dauerte 6 Stunden.

Für die Dicke der Thiolschicht erhält man gegen Luft einen Wert von 3,0 nm und gegen die wäßrige Umgebung 3,2 nm. Unter Berücksichtigung eines Meßfehlers von ca. ± 0,2 nm und möglichen Abweichungen des Brechungsindex der wäßrigen Subphase stimmt diese Dicke recht gut mit dem theoretischen Wert von ~2,8 nm überein.

Mit einer Belegung von ca. 100 % liegt hier ein dicht gepackter Biotinfilm vor.

Beispiel 7

Bindung von Streptavidin an den Biotin-Monolayer 2/Herstellung des Streptavidin-Monolayers 2

Die Bindung des Streptavidins kann auch online über die Verschiebung der (Flanke der) Resonanzkurve verfolgt werden, so daß auch kinetische Daten zugänglich sind.

Nimmt man für den Dickenzuwachs durch Adsorption einen monoexponentiellen Verlauf an, so erhält man für die reflektierte Intensität in Abhängigkeit von der Zeit:

$$I=I_\infty[1-\exp[-t/\tau)]$$

$I_\infty$: Intensität nach $t = \infty$

$\tau$ : Zeitkonstante

Die Zeitkonstante $\tau$, in der sich die Adsorption bis auf 1/e dem Sättigungswert genähert hat, beträgt in diesem Experiment ~13 Minuten.

Die resultierende Streptavidin-Schicht beträgt 0,8 nm, was einer sehr unvollständigen Belegung mit Protein entspricht. Die Homogenität der Schicht kann mit PSP-Mikroskopie kontrolliert werden mit dem Ergebnis, daß keine Unebenheiten festzustellen sind. Das Protein lagert sich demnach zwar als zu dünne, aber dennoch gleichmäßige Schicht (bzgl. der lateralen Auflösung von ~5 µm) an.

Mit einer Belegung von ca. 27 % liegt hier ein "verdünnter" Streptavidinfilm vor.

Beispiel 8

Herstellung des Biotin-Monolayers 3 aus der Biotinverbindung 3

Das biotinylierte Thiol mit Spacer aus Beispiel 3 wurde aus einer $1{,}25x10^{-4}$ M Lösung adsorbiert. Ansonsten waren die Präparationsbedingungen analog Beispiel 4.

Nach Beendigung der Proteinadsorption und Charakterisierung des Schichtsystems gegen die wäßrige Umgebung wurde die Probe gründlich mit 0,5 M NaCl-Lösung gespült und unter einem Stickstoffstrom getrocknet. Nach erneuter PSP-Spektroskopie gegen Luft konnte die Probe wieder wäßriger Umgebung ausgesetzt werden, um so eventuelle Veränderungen durch den Umgebungswechsel und das Spülen zu detektieren.

Für den biotinylierten Thiollayer ergab sich eine Dicke von 0,7 nm, sowohl gegen Luft als auch gegen die wäßrige Subphase. Dieser Wert ist bei weitem nicht mit dem theoretischen von -3,7 nm zu vergleichen, so daß die Moleküle wohl vereinzelt vorliegen und eine sehr unvollständige Schicht ausbilden.

Mit einer ca. 19 %igen Belegung liegt hier wiederum ein "verdünnter" Biotinfilm vor.

Beispiel 9

Bindung von Streptavidin an den Biotin-Monolayer 3/Herstellung des Streptavidin-Monolayers 3

Die Adsorption des Streptavidins erfolgte in diesem Fall außerordentlich schnell. Den gleichen monoexponentiellen Verlauf für die Dickenzunahme angenommen erhält man hier eine Zeitkonstante $\tau$ von nur 1 Minute.

Der Streptavidin-Layer erreicht insgesamt eine Dicke von 3,1 nm und erscheint in der PSP-Mikroskopie mit unspezifisch adsorbierten Proteinaggregaten oberflächig belegt. Ein Spülen mit der entsprechenden Subphase und Trocknen fügt dem Schichtsystem keinen Schaden zu, so daß die Dicke (gegen Luft) mit 3,0 nm annähernd konstant bleibt. Nach erneutem Befüllen der Küvette mit wäßriger Subphase erscheint der Film jetzt mikroskopisch als homogen.

Beispiel 10

Bindung eines biotinylierten Antikörpers an die Streptavidin-Monolayer 2 und 3

Streptavidin besitzt als tetrameres Protein vier Bindungsstellen für Biotin/biotinylierte Moleküle. Nach Anlagerung an den Fixierlayer sollten aufgrund geometrischer Überlegungen, in Richtung der wäßrigen Subphase zwei Bindungsstellen für weitere Erkennungsreaktionen zur Verfügung stehen. Dies wurde mit einem biotinylierten Antikörper gegen TSH, dem entsprechenden nicht modifizierten Antikörper (als Referenz) und dem dazugehörenden Antigen TSH als Probe überprüft. Die Durchführung der immunologischen Bestimmungsreaktion erfolgte wie in Beispiel 2 von EP-A 0 344 578 beschrieben.

Der biotinylierte Antikörper adsorbiert aus einer $1x10^{-6}$ M wäßrigen Lösung an verschieden präparierte Streptavidin-Layer. Die dabei erzielten Ergebnisse scheinen in einem starken Maße von der jeweiligen Dicke und damit der Pakkungsdichte der Streptavidin-Schicht abzuhängen.

So erhält man bei Adsorption des biotinylierten Antikörpers an einen dichtgepackten Steptavidin-Monolayer 3 aus Beispiel 9 von 3,0 nm, nur einen geringen Dickenzuwachs von 0,5 nm. Begleitet wird dieser Vorgang von einer anfänglichen Dickenabnahme, vermutlich durch das Lösen von nicht ausreichend fest fixiertem Streptavidin. Die anschließende Dickenzunahme erfolgt mit einer Zeitkonstante $\tau$ ~3 Minuten.

Adsorbiert man unter analogen Bedingungen den biotinylierten Antikörper jedoch an den Streptavidin-Monolayer 2 aus Beispiel 7 von 0,8 nm, so ergibt sich ein sehr viel stärkerer Effekt von insgesamt 5,1 nm Dickenzunahme. Bei genauerer Betrachtung der Kinetik erkennt man anfangs die geringe Dikkenabnahme und dann die sehr viel stärkere Adsorption. Die Kinetik muß hier wohl eher biexponentiell beschrieben werden. Für den ersten Zeitabschnitt ergibt sich als Zeitkonstante $\tau$ ~5 Minuten.

Der dichtgepackte Streptavidin-Monolayer 3 bindet den biotinylierten Antikörper langsamer als der "verdünnte" Streptavidin-Monolayer 2. Der "verdünnte" Streptavidin-Monolayer 2 hat außerdem eine wesentlich höhere Bindekapazität pro Flächeneinheit. Damit ist der erreichbare Meßeffekt pro Zeit bei dem "verdünnten" Streptavidinfilm wesentlich größer.

Tabelle

| Bindefilm | Dichte der Belegung | Hydrophiler Spacer an Biotin | Bindefähigkeit | Bindekinetik |
|---|---|---|---|---|
| Biotin-Monolayer 1[b)] | 30 % | nein | Bindet Streptavidin zu einem dichten Film | |
| Biotin-Monolayer 2[b)] | 100 % | nein | Bindet Streptavidin zu einem Film mit 27 % Belegung | τ = 13 min. |
| Biotin-Monolayer 3[b)] | 19 % | ja | Bindet Streptavidin zu einem dichtgepackten Film | τ = 1 min. |
| Streptavidin-Monolayer 2[b)] | 27 % | - | Bindet biotinylierten AK zu einem dichtgepackten Film | τ = 3 min. |
| Steptavidin-Monolayer 3[b)] | 100 % | - | Bindet biotinylierten AK zu einem zu ca. 10% belegten Film | τ = 5 min. |

[a)]: erfindungsgemäß [b)]: nicht erfindungsgemäß

Beispiel 11

**Synthese von Bis-dodecansäure-disulfid**

(Die Synthese wird unter Inertgas ($N_2$) durchgeführt)

a) 12-Mercapto-dodecansäuremethylester
2,3 g (0,1 mol) Natrium wird in 200 ml absolutem und entgastem Methanol gelöst. Die resultierende Lösung wird mit Eis gekühlt. Hierzu werden 7,1 ml (0,1 mol) Thioessigsäure, dann 14,4 g (50 mmol) Bromdodecansäure zugegeben und die Lösung 5 Std rückflußgekocht. Nach Abkühlung auf Raumtemperatur wird 15 ml conc. HCl zugefügt und 3 Std. unter Rückfluß gekocht. Anschließend werden nach Abkühlung 300 ml Ether zugegeben und die Etherphase 3 x mit $H_2O$, 1 x mit gesättigter NaCl-Lösung (je 100 ml) ausgeschüttelt. Nach Trocknung der organischen Phase über Natriumsulfat wird das Lösungsmittel abgezogen.
Ausbeute: 12,2 g
DC: $R_f$ = 0,86 (Kieselgel; Essigester/Petrolether 19/1 + 1% Essigsäure)
b) 12-Mercapto-dodecansäure
12,2 g des Produkts aus 11a) werden in 200 ml abs. und entgastem Methanol gelöst, mit 100 ml 1 N NaOH versetzt und 3 Std. unter Rückfluß gekocht. Nach Abkühlung auf Raumtemperatur wird das Reaktionsgemisch in ein Eisbad aus 400 ml Eiswasser, 20 ml conc. HCl und 600 ml Ether gekippt. Die Etherphase wird abgetrennt, 3 x mit je 300 ml $H_2O$, 1 x mit 100 ml gesättigter NaCl-Lösung ausgeschüttelt und über $Na_2SO_4$ getrocknet. Nach Abfiltration des Trockenmittels wird das Lösungsmittel abgezogen.
Ausbeute: 12,3 g
DC: $R_f$ = 0,68 (Kieselgel; Essigester/Petrolether 19/1 + 1% Essigsäure)
c) Bis-dodekansäure-disulfid
12,0 g des Produkts aus 11b) werden in 170 ml Ethanol suspendiert und unter kräftigem Rühren eine Lösung aus 6,3 g Jod in 200 ml Ethanol zugetropft. Die Zugabe wird abgebrochen, sobald eine gelbe Lösung entsteht, die sich nicht mehr entfärbt. Anschließend wird 550 ml Ether und 350 ml $H_2O$ zugegeben, die Wasserphase abgetrennt und nochmals mit Ether nachgewaschen. Die vereinigten Etherphasen werden mit gesättigter NaCl-Lösung geschüttelt und über $Na_2SO_4$ getrocknet. Der nach Filtration des Trockenmittels und Entfernung des Lösungsmittels verbleibende Rückstand wird aus Aceton umkristallisiert.
Ausbeute: 10 g (86 % d.Th.)
DC: $R_f$ = 0,57 (Kieselgel; Essigester/Petrolether 19/1 + 1% Essigsäure)

Beispiel 12

**Synthese von Bis-(biotinamido-3,6-dioxaoctyl)-dodecansäure-amid-disulfid (Biotin-Verbindung 4)**

1,38 g (3 mmol) des Produkts aus Beispiel 11c und 1.76 g (7 mmol) 2-Ethoxy-N-ethoxycarbonyl-1,2-dihydrochinolin (EEDQ) werden in einer Mischung aus 150 ml THF und 150 ml DMF gelöst, dann eine Lösung aus 2,25 g (6 mmol) Biotinoyl-1,8-diamino-3,6-dioxaoctan (Biotin-DADOO) in 40 ml DMF zugetropft. Das Reaktionsgemisch wird dann 3 Std lang bei 50°C erhitzt. Anschließend wird eingeengt und der Rückstand mit 100 ml Chloroform aufgenommen, 2 x mit je 100 ml $H_2O$ ausgeschüttelt. Nach Trocknung über $Na_2SO_4$ wird das Lösungsmittels abgezogen und der Rückstand über Kieselgel chromatographiert (Flash-Chromatographie, Laufmittel: Chloroform/Methanol 9/1).

DC: Rf = 0,31 (Kieselgel; Chloroform/Methanol 8/1)
MS: m/e = 1175 (pos. FAB)

Beispiel 13

**Synthese von S-(Biotinamido-3,6-dioxaoctyl)-dodecansäureamid-S'-dodecansäuredisulfid (Biotin-Verbindung 5)**

Ansatz identisch mit Beispiel 12
Ausbeute : 300 mg (9 % d.Th.)
DC: Rf = 0,05 (Kieselgel; Chloroform/Methanol 8/1)
MS: m/e = 817 (neg. FAB)

Beispiel 14

**Synthese von Biotinamido-3,6-dioxaoctyl-12-mercaptododekansäureamid (Biotin-Verbindung 6)**

(Die Synthese ist unter Inertgas durchzuführen)

300 mg (0,3 mmol) des Produkts aus Beispiel 12 und 0,5 g (3,3 mmol) Dithiothreitol (DTT) wird in 100 ml absolutem und entgastem Methanol gelöst und Inertgasatmosphäre 10 Std gerührt. Die Reaktionsmischung wird danach zur Trockene eingeengt, der Rückstand in Methylenchlorid aufgenommen und mit $H_2O$ ausgeschüttelt. Die organische Phase wird über $Na_2SO_4$ getrocknet, dann eingeengt und der Rückstand per Flash-Chromatographie (Kieselgel; Methylenchlorid/Ethanol 1/1) gereinigt.
DC: Rf = 0,27 (Kieselgel; Chloroform/Methanol 8/1)

Beispiel 15

**Desthiobiotin-N-hydroxy-succinimidester**

Zu einer Lösung aus 3 g (14 mmol) Desthiobiotin (Sigma) und 3,5 g (17 mmol) N,N'-Dicyclohexylcarbodiimid in einem Gemisch aus 40 ml DMF und 40 ml Dioxan gab man unter intensivem Rühren eine Lösung aus 1,8 g (15,5 mmol) N-Hydroxysuccinimid in 40 ml Dioxan. Man rührte 4 Stunden bei 20°C, wobei ein feiner weißer Niederschlag ausfiel, der nach beendeter Reaktion (DC-Kontrolle) abfiltriert wurde. Das Filtrat wurde im Vakuum eingeengt, mit wenig Ethylacetat/DMF 4/1 aufgenommen und bei 4°C 16 Stunden stehen gelassen. Dabei fiel weiterer Niederschlag aus, der abfiltriert wurde. Diesen Vorgang wiederholte man mehrfach, bis keine Ausfällung mehr zu beobachten war. Das eingeengte Filtrat wurde in wenig Ethanol aufgenommen und durch Zugabe von Diisopropylether das Produkt ausgefällt. Nach Filtration ließ man das Filtrat 16 Stunden bei 4°C stehen, wobei weiterer Niederschlag ausfiel und abfiltriert wurde. Nach Trocknen im Vakuum bei 50°C erhielt man ein weißes, feinkristallines Produkt.
Ausbeute: 1,6 g (37 %)
DC (Kieselgel 60): Eluens Ethylacetat/Methanol = 6/4 RF = 0,75

Beispiel 16

**D-Desthiobiotinoyl-1,8-diamino-3,6-dioxaoctan, Desthiobiotin-DADOO**

Zu einer Lösung von 7,5 ml (50 mmol) 1,8-Diamino-3,6-dioxaoctan in 50 ml Dioxan wurde eine Lösung aus 1,6 g (5 mmol) Desthiobiotin-N-hydroxysuccinimidester langsam zugetropft und das Reaktionsgemisch 16 Stunden gerührt. Nach Abschluß der Umsetzung wurde das Gemisch am Rotationsverdampfer zum öl eingedampft, mit Ethylacetat/Ether ein Teil des überschüssigen DADOO herausgewaschen und der Rest über Säulenchromatographie an Kieselgel aufgereinigt.
Kieselgel 60, Eluens Ethylacetat/Methanol 6/4 + 10% $NH_3$.
DC: gleiches Eluens, RF = 0,3
Ausbeute: 0,8 g (50 %)

Beispiel 17

**Bis-(Desthiobiotinamido-3,6-dioxaoctyl-)dodecansäureamiddisulfid**

Desthiobiotinverbindung 1

Eine Lösung von 1,6 g (5 mmol) Desthiobiotin-DADOO (6), 2,3 g (0,5 mmol) Bis-dodecansäure-disulfid (Beispiel 11c), 0,74 g (5,5 mmol) 1-Hydroxy- benzotriazol und 1,14 g (5,5 mmol) N,N'-Dicyclohexylcarbodiimid in 50 ml Dimethylformamid werden 24 Stunden bei 20°C gerührt. Nach beendeter Reaktion (DC-Kontrolle) wird das Reaktionsgemisch am Rotationsverdampfer zum Öl eingedampft und über Flash-Chromatographie an Kieselgel (Eluens: Chloroform/Methanol 9/1) gereinigt.
DC: Kieselgel 60, Eluens Chloroform/Methanol 9/1,
RF = 0,4
Ausbeute: 0,25 g (5%)

Beispiel 18

S-(Desthiobiotinamido-3,6-dioxaoctyl)-dodecansäureamid-S'-dodecansäuredisulfid

Desthiobiotinverbindung 2

Aus der Flash-Chromatographie des Reaktiongemisches aus Beispiel 17 erhielt man diese Verbindung als weißes amorphes Pulver.
DC: Kieselgel 60, Eluens Chloroform/Methanol 9/1,
RF = 0,3
Ausbeute: 0,8 g (20 %)

Beispiel 19

**12-Mercapto-(desthiobiotinamido-3,6-dioxaoctyl)-dodekansäure-amid**
**Desthiobiotinverbindung 3**

Eine Lösung aus 200 mg (0,2 mmol) der Verbindung aus Beispiel 17 und 2 g (13 mmol) Dithiothreitol in 50 ml Methanol wurde 36 Stunden unter Rückfluß erhitzt. Nach dem Abkühlen wurde das Lösungsmittel am Rotationsverdampfer abgezogen und der Rückstand in Chloroform aufgenommen. Anschließend wird so häufig mit Wasser ausgeschüttelt, bis im DC kein Dithiothreitol mehr nachweisbar ist. Die Chloroformphase wird über Natriumsulfat getrocknet und am Hochvakuum eingedampft. Die Aufreinigung erfolgte durch Flash-Chromatographie an Kieselgel, Eluens: Chloroform/Isopropanol 18/2.
DC: Kieselgel 60, Eluens Chloroform/Methanol 9/1, RF = 0,35 Ausbeute: 60 mg (30 %)

Beispiel 20

**Diphenylhydantoin-N-propionamido-3,6-dioxaoctyl-12-mercapto-dodecansäureamid**

Diphenylhydantoin-Verbindung

Analog Beispiel 16 erhält man aus Diphenylhydantoin-N-propionsäure (Cook et al., Res. Communications in Chemical Pathology and Pharmacology 5, (1973), S. 767) und 1,8-Di-amino-3,6-dioxaoctan Diphenylhydantoin-N-propionyl-DADOO. Aus Diphenylhydantoin-N-propionyl-DADOO und Bis-dodekansäure-disulfid gemäß Beispiel 11 c) erhält man analog Beispiel 13 das entsprechende gemischte Disulfid; nach Spaltung mit DTT Diphenylhydantoin-N-propionamido-3,6-dioxaoctyl-12-mercaptododecansäureamid.

Beispiel 21

**Synthese von Bis-(hydroxyphenylazo-benzoylamido-3,6-dioxaoctyl)-dodecansäureamid-disulfid [Bis-(HABA-DADOO)-dodecansäureamid-disulfid]**

Die Synthese erfolgt analog Beispiel 12 mit 1,4 g des Produkts aus Beispiel 11c und 2,4 g [2-(4-Hydroxyphenylazo)-benzoyl]-1,8-diamino-3,6-dioxaoctan (HABA-DADOO)
DC: Rf = 0,65 (Kieselgel; Essigester/Methanol 4/1)

Die Synthese von HABA-DADOO erfolgt analog Beispiel 16 aus HABA-OSu und DADOO (Diamino-3,6-dioxaoctan). HABA-OSu wird analog Beispiel 15 aus HABA und N-Hydroxysuccinimid hergestellt.
DC (HABA-DADOO): Rf = 0,52
(Kieselgel; Butanol/Eisessig/Wasser 10/3/5)
DC (HABA-OSu): Rf = 0,89
(Kieselgel; Butanol/Eisessig/Wasser 10/3/5)

Beispiel 22

**Synthese von (Hydroxyphenylazo-benzoylamido-3,6-dioxaoctyl)-12-mercaptododecansäureamid.**

(Die Synthese wird unter Inertgas durchgeführt)

Die Synthese erfolgt analog Beispiel 14 mit 200 mg des Produkts aus Beispiel 21.
DC: Rf = 0,68 (Kieselgel; Essigester/Methanol 4/1)

Beispiel 23

**Herstellung von Biotin-Monolayern aus der Biotinverbindung 5 und Bis(11-hydroxyundecyl)disulfid**

Die Beschichtung erfolgt analog Beispiel 4. Als feste Träger dienen Objektträger aus Hochindexglas LA SF N 9, die mit Gold (50 nm) bedampft wurden.

Zur Beschichtung wurde die unsymmetrische, ein Biotinmolekül tragende Disulfid Biotin-Verbindung 5 (Beispiel 13) und das symmetrische kein Biotin-Molekül tragende Bis(11-hydroxyundecyl)disulfid (Zwischenstufe in Bsp.2) eingesetzt. Beide Verbindungen tragen jeweils 2 Spacer $(CH_2)_{11}$.

| Mischungen | Molenbruch Biotinverbindung 5 | Molenbruch Biotin bezogen auf die Spacer | Belegungsgrad [ca. %] |
|---|---|---|---|
| "Mischung" 1 (reine Biotinverbindung 5) | 1.0 | 0.5 | 50 |
| Mischung 2 | 0.6 | 0.3 | 30 |
| Mischung 3 | 0.4 | 0.2 | 20 |
| Mischung 4 | 0.2 | 0.1 | 10 |
| "Mischung" 5 (reines Bis) 11-hydroxyundecyl)disulfid | 0.0 | 0.0 | 0 |

Die Gesamtkonzentration der Komponenten in den einzelnen Mischungen ist jeweils $5 * 10^{-4}$ mol/l in Ethanol.

Unter einer Argonschutzatmosphäre wird der Objektträger 6 Stunden lang mit der Lösung inkubiert, anschließend mit reinem Ethanol gespült und in einem Argonstrom getrocknet. Analog Beispiel 4 wird die Dicke des adsorbierten Biotin-haltigen Monolayers bestimmt.

Unter der Annahme eines Brechungsindex von 1.45 ergeben sich die in Fig. 2 gezeigten Dicken des adsorbierten Monolayers. Für die "Mischung" 1; d.h. den reinen Monolayer aus der Biotinverbindung 5 erhält man eine Dicke von 260 nm. Vergleicht man mit dem theoretischen Wert von ca. 250 nm, der aus dem Anteil von ca. 360 nm für den Biotin tragenden Teil des Disulfids und ca. 130 nm für den nicht Biotin tragenden Anteil folgt, ist die Oberfläche mit einem dichten Monolayer belegt. Da nur die Hälfte der Spacermoleküle mit einem Biotin verknüpft sind, ist die Belegung mit Biotin 50%.

Für die "Mischung" 5 erhält man eine dichte Belegung mit dem Biotin-freien Disulfid; für die Mischungen 2 - 4 abnehmende Dicken, die auf einen dem Mischungsverhältnis in der Inkubationslösung entsprechenden Anteil der Biotinverbindung 5 schließen lassen.

Beispiel 24

**Bindung von Streptavidin an die Biotin tragenden Monolayer aus Beispiel 23**

Analog Beispiel 5 werden die in Beispiel 23 erhaltenen Biotin tragenden Monolayer mit Streptavidin inkubiert. Die Sättigungswerte für den Dickenzuwachs durch die Streptavidinbindung sind in Fig. 2 gezeigt. Für den Molenbruch von 0.1 - 0.5 an Biotin beobachtet man eine sehr hohe Strepavidinbindefähigkeit. Es resultieren dichte Streptavidinfilme mit einer Belegung zwischen 67% und 100%. Die Bindekinetik ist mit Halbwertszeiten von 1-2 min. sehr schnell.

Beispiel 25

**Synthese von 1-tert.Butyloxycarbonyl-1,8-diamino-dioxaoctan, (mono-BOC-DADOO)**

Eine Lösung von 142 g (1 mol) 1,8-Diamino-3,6-dioxaoctan (DADOO) in 900 ml Dioxan/Wasser (1/1 v/v) wird unter rühren langsam mit einer Lösung von 109 g (0,5 mol) Di-tert.butyldicarbonat in 450 ml Dioxan versetzt. Nach der Zugabe wird das Gemisch noch 1,5 h bei 20°C gerührt, anschließend das Lösungsmittel abdestilliert und der Rückstand in 1 1 Ethylacetat/Wasser (1/1 v/v) aufgenommen. Nach dem Abtrennen der wäßrigen Phase extrahiert man die organische Phase zweimal mit je 100 ml 0,1 N HCl. Die wäßrigen Phasen werden vereinigt, der pH-Wert mit verdünnter Natronlauge auf pH 9 bis 10 gestellt und die Lösung einer Flüssig-Flüssig-Extraktion im Perforator unterworfen. Nach 8-stündigem Extrahieren mit 750 ml Ethylacetat wird das Lösungsmittel entfernt und der Rückstand am Hochvakuum getrocknet.
Ausbeute: 32 g (26 %)
DC: Kieselgel 60,
Eluens Butylacetat/Wasser/Ammoniumhydroxid = 30/15/5,
RF = 0,45

Beispiel 26

**Synthese von 1-(Biotin-aminocapronsäure)-(1,8-diamino-4,6-dioxaoctan)-amid, (Biotin-X-DADOO)**

Eine Lösung von 0,9 g (2 mmol) D-Biotinoyl-aminocapronsäure N-hydroxysuccinimidester (Boehringer Mannheim, Best.Nr. 1003933) und 0,5 g (2 mmol) mono-BOC-DADOO in 10 ml Dioxan und 10 ml 0,1 mol/l Kaliumphosphatpuffer pH 8,5 werden ca. 2 Stunden bei 20°C gerührt. Nach beendeter Reaktion (DC-Kontrolle: Kieselgel 60, Eluens Ethylacetat/Methanol = 3/7, RF = 0,6) wird das Lösungsmittel im Vakuum abgedampft und der Rückstand mit 1 ml Trifluoressigsäure versetzt. Man rührt ca. 30 min bis zur vollständigen Abspaltung der BOC-Gruppe. Anschließend dampft man die Trifluoressigsäure im Vakuum ab, versetzt den Rückstand mit 5 ml Ethylacetat, filtriert von Ungelöstem ab und dampft das Filtrat zur Trockene ein.
Ausbeute: 0,96 g (98 %)
DC: Kieselgel 60, Eluens Ethylacetat/Methanol = 2:8, RF = 0,2

Beispiel 27

**Synthese von S-Acetyl-mercaptopropionsäure**

Zu 10,6 g (100 mmol) Mercaptopropionsäure wird bei 20°C langsam 8,6 g (110 mmol) Acetylchlorid zugetropft. Nach beendeter Zugabe wird 10 min auf 100°C erhitzt. Das Reaktionsgemisch wird einer Vakuumdestillation unterzogen und das Produkt bei 0,4 bar und 105°C rein gewonnen.
Ausbeute: 5,8 g (36 %)
1-H-NMR ($CDCl_3$): $\delta$ (ppm) = 2,3 (s, 3H), 2,7 (t, 2H), 3,1 (t, 2H).

Beispiel 28

**Synthese von N-Succinimidyl-S-acetylthiopropionat, (SATP)**

16,2 g (0,1 mol) S-Acetyl-mercaptopropionsäure, 12,7 g (0,11 mol) N-Hydroxysuccinimid und 22,7 g (0,11 mol) Dicyclohexylcarbodiimid werden in 0,4 1 absolutem Ethylacetat 16 h bei 20°C gerührt. Ausgefallener Niederschlag wird abfiltriert und das Filtrat im Vakuum eingedampft. Der ölige Rückstand wird in wenig Ethylacetat aufgenommen und gekühlt. Dabei fällt weiterer Niederschlag aus, der verworfen wird. Dieser Vorgang wird zweimal wiederholt. Aus dem letzten Filtrat erhält man nach Eindampfen 13 g (50 %) SATP.
1H-NMR ($CDCl_3$): $\delta$ (ppm) = 2,3 (s, 3H), 2,8 (s,4H), 2,9 (m, 2H), 3,1 (m, 2H).

Beispiel 29

**Synthese von Biotin-aminocapronsäure-amidodioxaoctyl-mercaptopropionsäure-amid, (Biotin-Verbindung 7)**

Eine Lösung aus 0,96 g (2 mmol) Biotin-X-DADOO (aus Beispiel 26) und 0,5 g (2 mmol) SATP (aus Beispiel 28) werden in 20 ml Dioxan und 20 ml 0,1 mol/l Kaliumphosphatpuffer pH 8,5 bei 20°C 2 h gerührt. Anschließend wird die

Lösung zur Trockene eingedampft, der Rückstand mit 2 ml Trifluoressigsäure aufgenommen und unter Inertgas 0,5 h bei 20°C gerührt. Die Aufreinigung erfolgt durch Flash-Chromatographie an Kieselgel (Eluens: Ethylacetat/Methanol = 3:7).
Ausbeute: 150 mg (13 %)
DC: Kieselgel 60, Ethylacetat/Methanol = 3/7,
RF = 0,35

Beispiel 30

**Synthese von Biotinamido-3,6-dioxaoctyl-S-acetyl-mercaptopropionsäureamid, (Biotin-DADOO-SATP)**

1 g (2,7 mmol) Biotin-DADOO gelöst in 40 ml 0,1 mol/l Kaliumphosphatpuffer pH 7,0 wird langsam mit einer Lösung aus 1,4 g (5,35 mmol) SATP (aus Beispiel 28) in 40 ml Dioxan versetzt. Während der Zugabe muß der pH-Wert kontinuierlich mit 0,1 mol/l Kaliumphosphatpuffer auf pH 7,0 nachgestellt werden. Nach beendeter Zugabe wird noch 10 min gerührt und anschliessend zur Trockene eingeengt. Das Rohprodukt kann ohne weitere Aufreinigung in die folgende Stufe eingesetzt werden.
DC: Kieselgel 60, Eluens Ethylacetat/Methanol = 3,5/7,5,
RF = 0,35.

Beispiel 31

**Synthese von Bis-(biotinamido-3,6-dioxaoctyl)-mercaptopropionsäureamid-disulfid, (Biotin-Verbindung 8)**

1,6 g des Rohproduktes aus Beispiel 30 wird in 100 ml Stickstoff-gesättigtem 0,5 mol/l Kaliumphosphatpuffer pH 7,5 gelöst und mit 5,4 ml 1 mol/l methanolischer HydroxylaminLösung versetzt. Es wird 2 h bei 20°C gerührt, anschließend im Vakuum zur Trockene eingedampft und über Flash-Chromatographie an Kieselgel (Ethylacetat/Methanol = 3/7) aufgereinigt.
Ausbeute: 150 mg (6 %)
DC: Kieselgel 60, Eluens Ethylacetat/Methanol = 3/7,
RF = 0,35

Beispiel 32

**Synthese von Biotinamido-3,6-dioxaoctyl-S-acetylmercaptoessigsäureamid, (Biotin-DADOO-SATA)**

Die Herstellung erfolgt analog Beispiel 30 aus 187 mg (0,8 mmol) N-Succinimidyl-S-acetyl-thioacetat (SATA) (Boehringer Mannheim, Best.Nr. 1081765) und 300 mg (0,8 mmol) Biotin-DADOO.
Ausbeute: 109 mg (49 %)
DC: Kieselgel 60, Eluens Ethylacetat/Methanol = 6,5/3,5
RF = 0,35

Beispiel 33

**Synthese von Bis-(biotinamido-3,6-dioxaoctyl)-mercaptoessigsäureamid-disulfid, (Biotin-Verbindung 9)**

Die Herstellung erfolgt analog Beispiel 31 aus 100 mg (0,2 mmol) Biotin-DADOO-SATA und 0,25 ml 1 mol/l methanolischer Hydroxylaminlösung.
Ausbeute: 55 mg (60 %).
DC: Kieselgel 60, Eluens Ethylacetat/Methanol = 3/7,
RF = 0,35

**Patentansprüche**

1. Bindematrix, enthaltend ein Trägermaterial und einen daran über Ankergruppen adsorbierten Festphasen-Reaktanden, der mit mindestens einem freien Reaktionspartner bindefähig ist, **dadurch gekennzeichnet**, daß der Festphasen-Reaktand, der mit der Ankergruppe jeweils über ein flexibles Spacermolekül verknüpft ist, eine verdünnte und lateral homogene Bindeschicht auf der Oberfläche des Trägermaterials bildet, und daß die Bindeschicht weiterhin Spacermoleküle enthält, die mit Ankergruppen versehen sind, aber nicht mit Festphasen-Reaktanden verknüpft sind.

**2.** Bindematrix nach Anspruch 1,
**dadurch gekennzeichnet**, daß die Belegung des Festphasen-Reaktanden auf der Oberfläche des Trägermaterials von 0,1 bis 90 % der maximalen Belegung ist.

**3.** Bindematrix nach Anspruch 2,
**dadurch gekennzeichnet**, daß die Belegung des Festphasen-Reaktanden auf der Oberfläche des Trägermaterials von 0,5 bis 70 % der maximalen Belegung ist.

**4.** Bindematrix nach Anspruch 3,
**dadurch gekennzeichnet**, daß die Belegung des Festphasen-Reaktanden auf der Oberfläche des Trägermaterials von 1 bis 40 % der maximalen Belegung ist.

**5.** Bindematrix nach einem der Ansprüche 1 bis 4,
**dadurch gekennzeichnet**, daß das Trägermaterial eine Metall-, Metalloxid- oder Glasoberfläche aufweist.

**6.** Bindematrix nach Anspruch 5,
**dadurch gekennzeichnet**, daß das Trägermaterial eine Gold-, Silber- oder Palladiumoberfläche aufweist und die Ankergruppe eine Thiol-, Disulfid- oder eine Phosphingruppe ist.

**7.** Bindematrix nach Anspruch 1,
**dadurch gekennzeichnet**, daß das flexible Spacermolekül mindestens eine Alkylengruppe der Formel $(CH_2)_n$ enthält, worin n eine natürliche Zahl zwischen 1 und 30 ist.

**8.** Bindematrix nach Anspruch 1 oder 7 ,
**dadurch gekennzeichnet**, daß ein Spacermolekül mit zwei oder mehreren Molekülen des Festphasen-Reaktanden verknüpft ist.

**9.** Bindematrix nach Anspruch 8,
**dadurch gekennzeichnet**, daß das Spacermolekül aus Cystamin gebildet ist.

**10.** Bindematrix nach Anspruch 1 oder 7,
**dadurch gekennzeichnet**, daß sich zwischen dem Spacermolekül und dem Festphasen-Reaktanden eine hydrophile Linkergruppe befindet.

**11.** Bindematrix nach Anspruch 10,
**dadurch gekennzeichnet**, daß die hydrophile Linkergruppe eine oder mehrere Oxyethylengruppen enthält.

**12.** Bindematrix nach Anspruch 11,
**dadurch gekennzeichnet**, daß die hydrophile Linkergruppe durch ein Amin- oder Hydroxyl-terminiertes Polyethylenoxid gebildet wird.

**13.** Bindematrix nach Anspruch 12,
**dadurch gekennzeichnet**, daß die hydrophile Linkergruppe aus 1,8-Diamino-3,6-dioxaoktan gebildet ist.

**14.** Bindematrix nach einem der vorhergehenden Ansprüche,
**dadurch gekennzeichnet**, daß der Festphasen-Reaktand ein mit einem Antikörper bindefähiges Antigen oder Hapten ist.

**15.** Bindematrix nach einem der Ansprüche 1 bis 13,
**dadurch gekennzeichnet**, daß der Festphasen-Reaktand Biotin ist.

**16.** Bindematrix nach Anspruch 1 oder 7,
**dadurch gekennzeichnet**, daß der Festphasen-Reaktand aus einer inneren und einer äußeren Komponente besteht, wobei die äußere Komponente mit mindestens einem freien Reaktionspartner bindefähig ist.

**17.** Bindematrix nach Anspruch 16,
**dadurch gekennzeichnet**, daß die innere Komponente des Festphasen-Reaktanden eine unverdünnte Schicht auf der Oberfläche des Trägermaterials bildet und die äußere Komponente an die innere Komponente durch Affinitätsbindung gekoppelt ist.

**18.** Bindematrix nach Anspruch 17,
**dadurch gekennzeichnet**, daß die innere Komponente Biotin und die äußere Komponente Streptavidin ist.

**19.** Verfahren zur Bestimmung eines Analyten in einer Probelösung mittels einer spezifischen Bindungsreaktion zwischen mindestens zwei bioaffinen Reaktanden, von denen einer an eine Festphase gekuppelt vorliegt und der oder die anderen Reaktionspartner frei sind,
**dadurch gekennzeichnet**, daß man einen Festphasen-Reaktanden verwendet, der Bestandteil einer Bindematrix nach einem der Ansprüche 1 bis 18 ist.

**20.** Verfahren nach Anspruch 19,
**dadurch gekennzeichnet**, daß man die spezifische Bindungsreaktion optisch, elektronisch, über die Wärmetönung oder die Massenbilanz bestimmt.

**21.** Verfahren nach Anspruch 19,
**dadurch gekennzeichnet**, daß man die spezifische Bindungsreaktion durch reflexionsoptische Techniken bestimmt.

**22.** Verfahren nach Anspruch 21,
**dadurch gekennzeichnet**, daß man die spezifische Bindungsreaktion plasmonenspektroskopisch bestimmt.

**23.** Verfahren nach Anspruch 19,
**dadurch gekennzeichnet**, daß man die spezifische Bindungsreaktion potentiometrisch oder amperometrisch bestimmt.

**24.** Verfahren nach Anspruch 19,
**dadurch gekennzeichnet**, daß man die spezifische Bindungsreaktion über die elektrische Leitfähigkeit oder Kapazitätsänderung bestimmt.

**25.** Verfahren zur Herstellung einer Bindematrix nach einem der Ansprüche 1 bis 18,
**dadurch gekennzeichnet**, daß man das Trägermaterial mit einer Reaktionslösung inkubiert, worin Moleküle enthalten sind, welche die an das Trägermaterial adsorbierte Bindeschicht bilden.

## Claims

**1.** Binding matrix containing a carrier material and a solid phase reactant which is adsorbed to this via anchor groups that is capable of binding to at least one free reaction partner,
**wherein**
the solid phase reactant which is linked to the anchor group via a flexible spacer molecule forms a dilute and laterally homogeneous binding layer on the surface of the carrier material and wherein the binding layer additionally contains spacer molecules which are provided with anchor groups but not with the solid phase reactant.

**2.** Binding matrix as claimed in claim 1,
**wherein**
the coverage of the solid phase reactant on the surface of the carrier material is 0.1 to 90 % of the maximum coverage.

**3.** Binding matrix as claimed in claim 2,
**wherein**
the coverage of the solid phase reactant on the surface of the carrier material is 0.5 to 70 % of the maximum coverage.

**4.** Binding matrix as claimed in claim 3,

**wherein**
the coverage of the solid phase reactant on the surface of the carrier material is 1 to 40 % of the maximum coverage.

5. Binding matrix as claimed in one of the claims 1 to 4,
**wherein**
the carrier material has a metal, metal oxide or glass surface.

6. Binding matrix as claimed in claim 5,
**wherein**
the carrier material has a gold, silver or palladium surface and the anchor group is a thiol group, disulfide group or a phosphine group.

7. Binding matrix as claimed in claim 1,
**wherein**
the flexible spacer molecule contains at least one alkylene group having the formula $(CH_2)_n$ in which n is a natural number between 1 and 30.

8. Binding matrix as claimed in claim 1 or 7,
**wherein**
a spacer molecule is linked to two or several molecules of the solid phase reactant.

9. Binding matrix as claimed in claim 8,
**wherein**
the spacer molecule is formed from cystamine.

10. Binding matrix as claimed in claim 1 or 7,
**wherein**
a hydrophilic linker group is located between the spacer molecule and the solid phase reactant.

11. Binding matrix as claimed in claim 10,
**wherein**
the hydrophilic linker group contains one or several oxyethylene groups.

12. Binding matrix as claimed in claim 11,
**wherein**
the hydrophilic linker group is formed by an amino terminal or hydroxyl-terminal polyethylene oxide.

13. Binding matrix as claimed in claim 12,
**wherein**
the hydrophilic linker group is formed from 1,8-diamino-3,6-dioxaoctane.

14. Binding matrix as claimed in one of the previous claims,
**wherein**
the solid phase reactant is an antigen or hapten capable of binding to an antibody.

15. Binding matrix as claimed in one of the claims 1 to 13,
**wherein**
the solid phase reactant is biotin.

16. Binding matrix as claimed in claim 1 or 7,
**wherein**
the solid phase reactant is comprised of an inner and an outer component whereby the outer component is capable of binding to at least one free reaction partner.

17. Binding matrix as claimed in claim 16,
**wherein**
the inner component of the solid phase reactant forms an undiluted layer on the surface of the carrier material and the outer component is coupled to the inner component by affinity binding.

**18.** Binding matrix as claimed in claim 17,
**wherein**
the inner component is biotin and the outer component is streptavidin.

**19.** Method for the determination of an analyte in a sample solution by means of a specific binding reaction between at least two reactants with bioaffinity one of which is present coupled to a solid phase and the other reaction partner or reaction partners are free,
**wherein**
a solid phase reactant is used which is a component of a binding matrix as claimed in one of the claims 1 to 18.

**20.** Method as claimed in claim 19,
**wherein**
the specific binding reaction is determined optically, electronically, via heat tonality or mass analysis.

**21.** Method as claimed in claim 19,
**wherein**
the specific binding reaction is determined by optical reflection techniques.

**22.** Method as claimed in claim 21,
**wherein**
the specific binding reaction is determind by plasmon spectroscopy.

**23.** Method as claimed in claim 19,
**wherein**
the specific binding reaction is determined potentiometrically or amperometrically.

**24.** Method as claimed in claim 19,
**wherein**
the specific binding reaction is determined by means of the electrical conductivity or change in capacitance.

**25.** Process for the production of a binding matrix as claimed in one of the claims 1 to 18,
**wherein**
the carrier material is incubated with a reaction solution which contains molecules which form the binding layer adsorbed to the carrier material.

## Revendications

1. Matrice de liaison contenant un matériau support et un corps réagissant en phase solide adsorbé sur celui-ci par l'intermédiaire de groupes d'ancrage, qui est capable de se lier à au moins un partenaire de réaction libre, caractérisée en ce que le corps réagissant en phase solide, qui est lié au groupe d'ancrage dans chaque cas par l'intermédiaire d'une molécule espaceur flexible, forme une couche de liaison diluée et latéralement homogène sur la surface du matériau support et en ce que la couche de liaison contient en outre des molécules espaceurs qui sont munies de groupes d'ancrage mais qui ne sont pas liées à un corps réagissant en phase solide.

2. Matrice de liaison selon la revendication 1, caractérisée en ce que le revêtement de la surface du matériau support par le corps réagissant en phase solide représente 0,1 à 90% du revêtement maximum.

3. Matrice de liaison selon la revendication 2, caractérisée en ce que le revêtement de la surface du matériau support par le corps réagissant en phase solide représente 0,5 à 70% du revêtement maximum. _

4. Matrice de liaison selon la revendication 3, caractérisée en ce que le revêtement de la surface du matériau support par le corps réagissant en phase solide représente 1 à 40% du revêtement maximum.

5. Matrice de liaison selon l'une des revendications 1 à 4, caractérisée en ce que le matériau support présente une surface métallique, d'oxyde métallique ou de verre.

6. Matrice de liaison selon la revendication 5, caractérisée en ce que le matériau support présente une surface d'or,

d'argent ou de palladium et le groupe d'ancrage est un groupe thiol, disulfure ou phosphine.

**7.** Matrice de liaison selon la revendication 1, caractérisée en ce que la molécule espaceur flexible contient au moins un groupe alkylène de formule $(CH_2)_n$ où n est un nombre naturel entre 1 et 30.

**8.** Matrice de liaison selon la revendication 1 ou 7, caractérisée en ce qu'une molécule espaceur est liée à deux ou plusieurs molécules du corps réagissant en phase solide.

**9.** Matrice de liaison selon la revendication 8, caractérisée en ce que la molécule espaceur est formée de cystamine.

**10.** Matrice de liaison selon la revendication 1 ou 7, caractérisée en ce qu'un groupe lieur hydrophile se trouve entre la molécule espaceur et le corps réagissant en phase solide.

**11.** Matrice de liaison selon la revendication 10, caractérisée en ce que le groupe lieur hydrophile contient un ou plusieurs groupes oxyéthylène.

**12.** Matrice de liaison selon la revendication 11, caractérisée en ce que le groupe lieur hydrophile est formé par un poly(oxyde d'éthylène) terminé par amine ou hydroxyle.

**13.** Matrice de liaison selon la revendication 12, caractérisée en ce que le groupe lieur hydrophile est formé de 1,8-diamino-3,6-dioxaoctane.

**14.** Matrice de liaison selon l'une des revendications précédentes, caractérisée en ce que le corps réagissant en phase solide est un antigène ou haptène capable de se lier à un anticorps.

**15.** Matrice de liaison selon l'une des revendications 1 à 13, caractérisée en ce que le corps réagissant en phase solide est la biotine.

**16.** Matrice de liaison selon la revendication 1 ou 7, caractérisée en ce que le corps réagissant en phase solide consiste en un composant interne et en un composant externe, le composant externe étant capable de se lier à au moins un partenaire de réaction libre.

**17.** Matrice de liaison selon la revendication 16, caractérisée en ce que le composant interne du corps réagissant en phase solide forme une couche non diluée sur la surface du matériau support et le composant externe est couplé au composant interne par liaison par affinité.

**18.** Matrice de liaison selon la revendication 17, caractérisée en ce que le composant interne est la biotine et le composant externe est la streptavidine.

**19.** Procédé pour déterminer un analyte dans une solution échantillon au moyen d'une réaction de liaison spécifique entre au moins deux corps réagissants à bioaffinité, parmi lesquels l'un est présent couplé à une phase solide et le ou les autres partenaires de réaction sont libres, caractérisé en ce que l'on utilise un corps réagissant en phase solide qui fait partie d'une matrice de liaison selon l'une des revendications 1 à 18.

**20.** Procédé selon la revendication 19, caractérisé en ce que l'on détermine la réaction de liaison spécifique par voie optique, électronique, par l'intermédiaire de la chaleur de réaction ou du bilan massique.

**21.** Procédé selon la revendication 19, caractérisé en ce que l'on détermine la réaction de liaison spécifique par des techniques optiques de réflexion.

**22.** Procédé selon la revendication 21, caractérisé en ce que l'on détermine la réaction de liaison spécifique par spectroscopie plasmonique.

**23.** Procédé selon la revendication 19, caractérisé en ce que l'on détermine la réaction de liaison spécifique par potentiométrie ou ampérométrie.

**24.** Procédé selon la revendication 19, caractérisé en ce que l'on détermine la réaction de liaison spécifique par le biais de la conductivité électrique ou d'une modification de la capacité.

**25.** Procédé pour préparer une matrice de liaison selon l'une des revendications 1 à 18, caractérisé en ce que l'on incube le matériau support avec une solution réactionnelle dans laquelle sont contenues des molécules qui forment la couche de liaison adsorbée sur le matériau support.

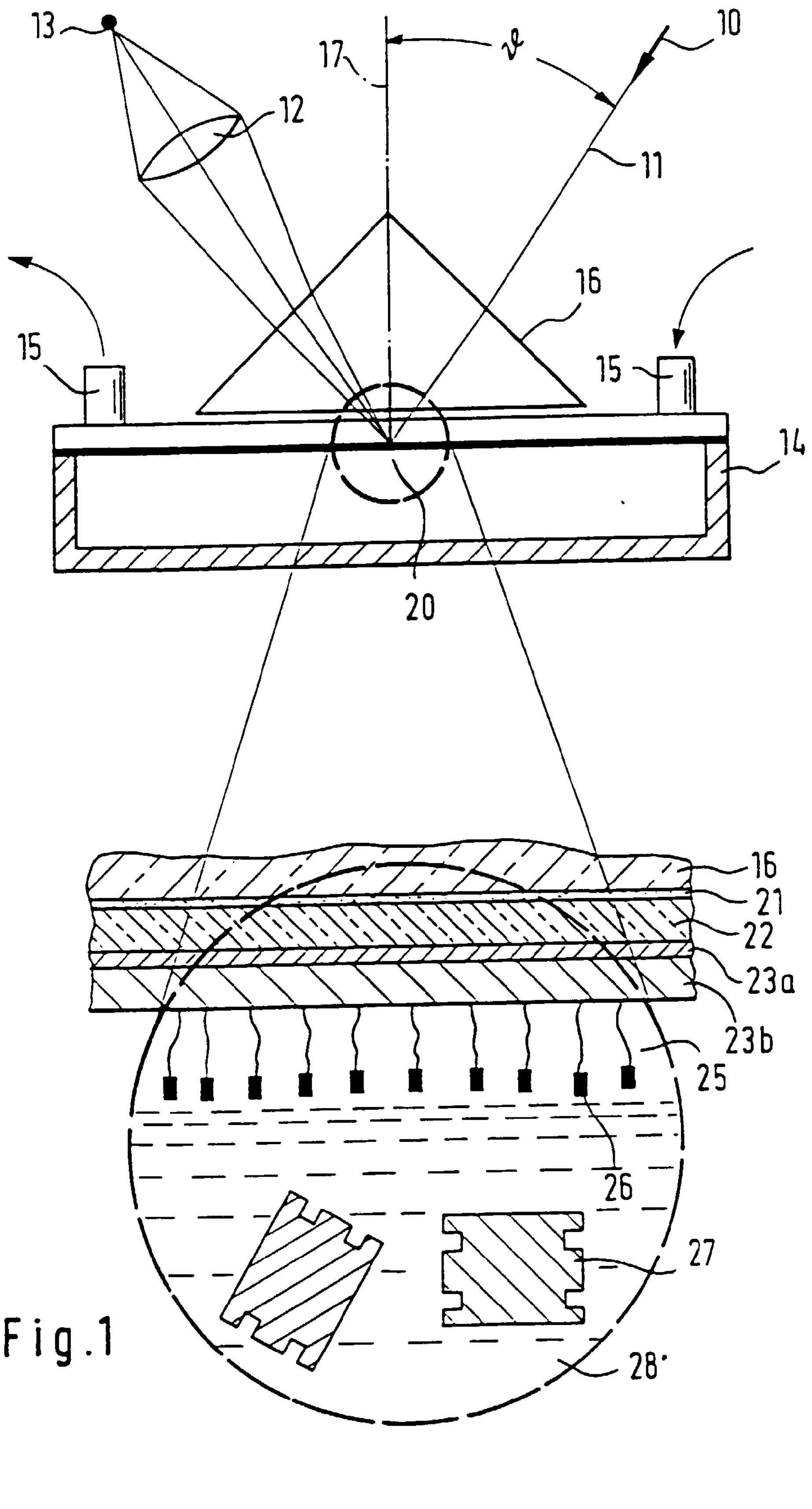


Fig.1

Fig. 2

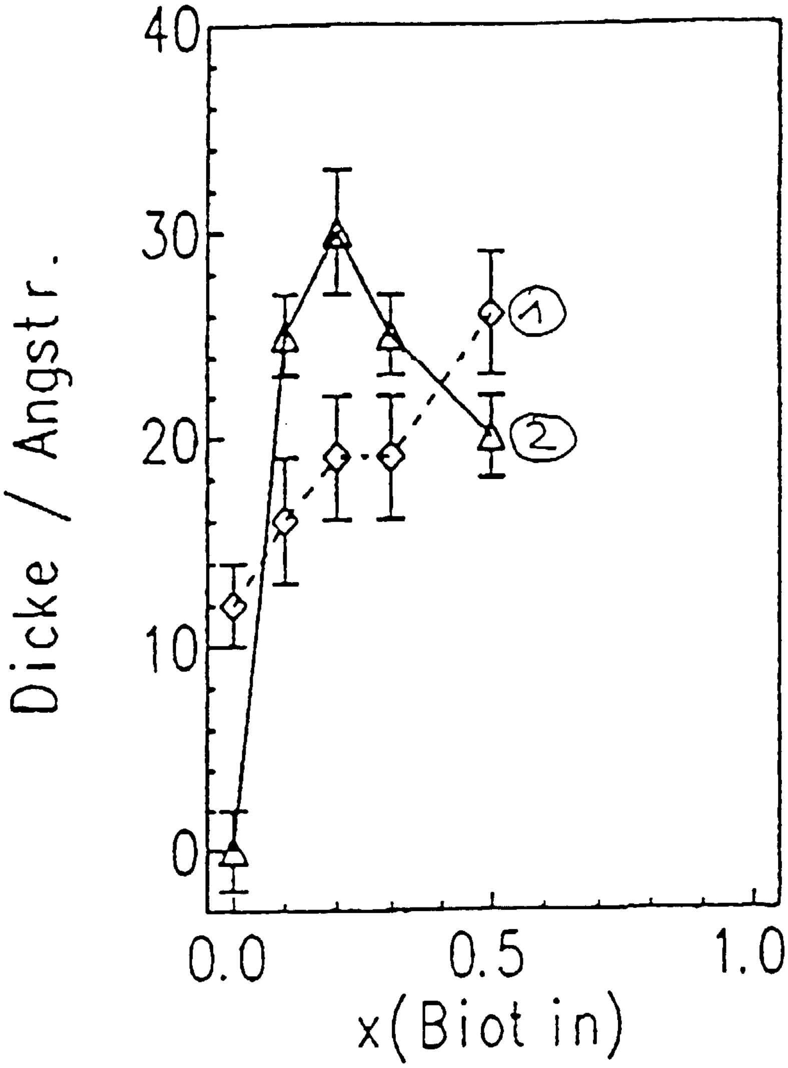

Fig. 3a

Biotinverbindung 1

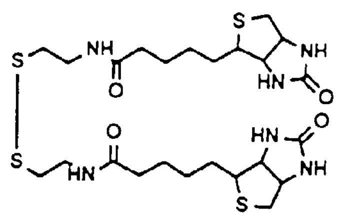

Biotinverbindung 2

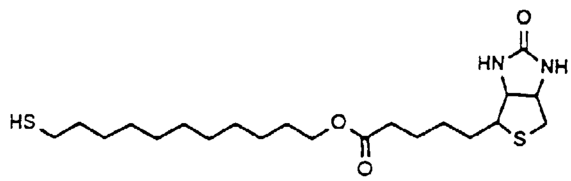

Biotinverbindung 3

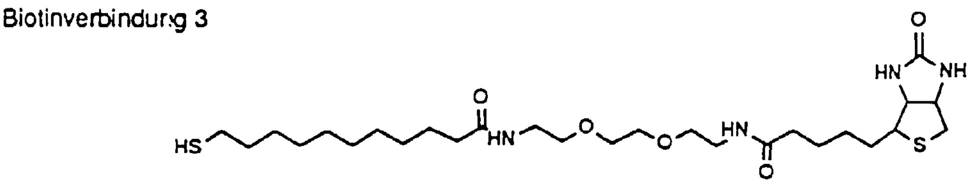

Biotinverbindung 4

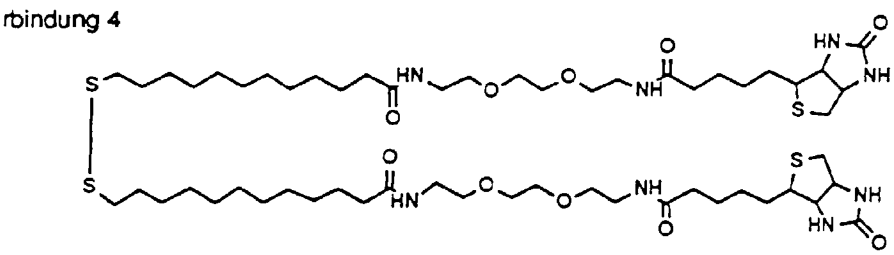

Biotinverbindung 5

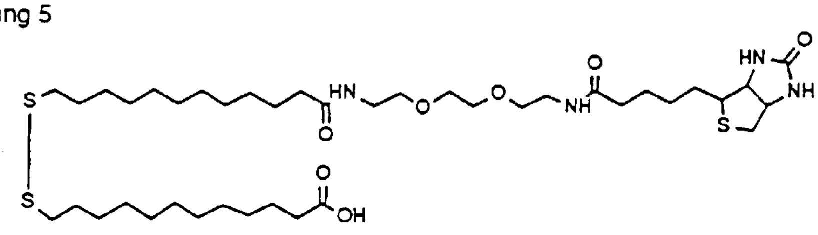

Biotinverbindung 6

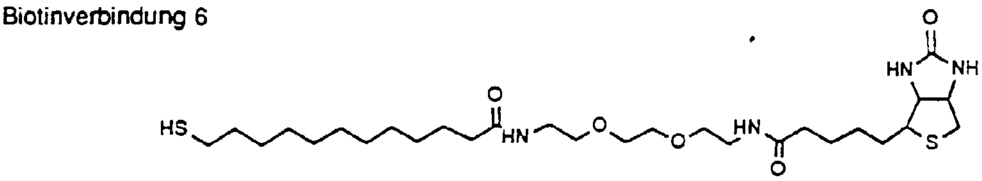

Fig. 3b

Desthioverbindung 1

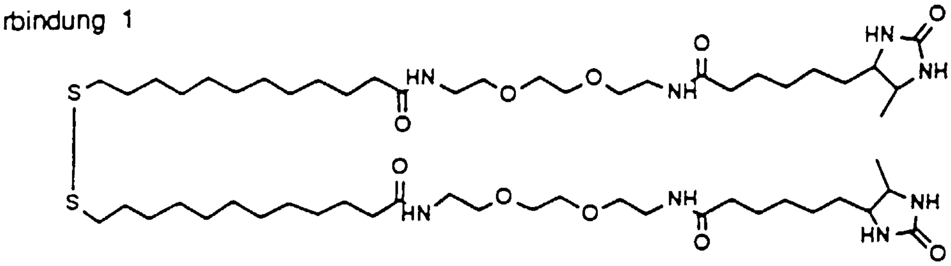

Desthioverbindung 2

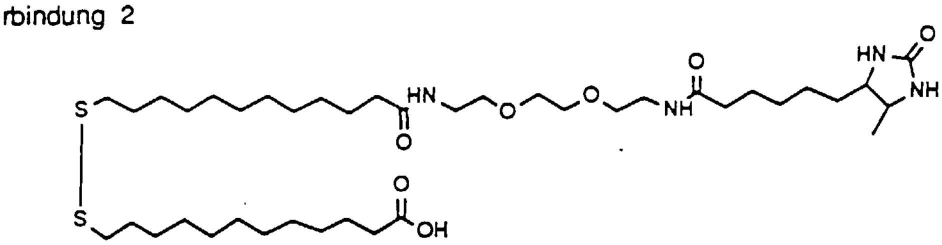

Desthioverbindung 3

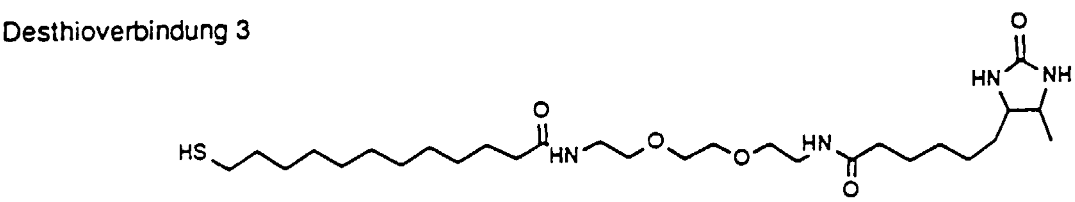

Biotinverbindung 7

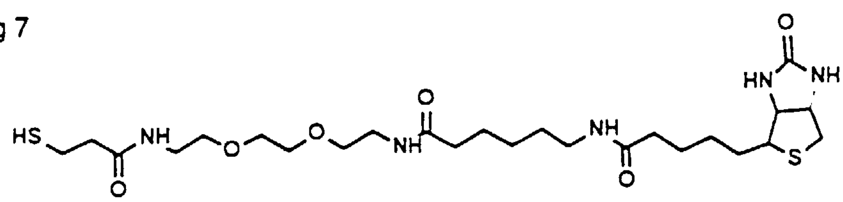

Biotinverbindung 8

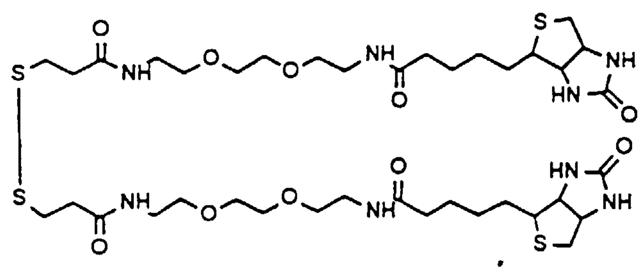

Fig. 3c

Biotinverbindung 9

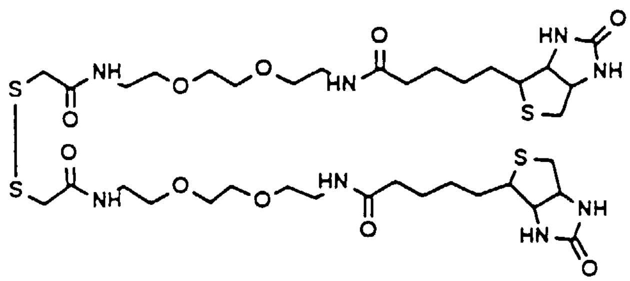

Diphenylhydantoinverbindung